# EUROPEAN PATENT SPECIFICATION

(11) **EP 3 346 015 B1**
(45) Date of publication and mention of the grant of the patent: **11.05.2022**
(21) Application number: 16842012.3
(22) Date of filing: 02.09.2016
(51) Int. Cl.: C12Q 1/68, C12N 15/00, G01N 30/88

(54) **PROGNOSIS METHOD FOR RENAL CELL CANCER**
PROGNOSEVERFAHREN FÜR NIERENZELLKARZINOM
PROCÉDÉ DE PRONOSTIC POUR LE CANCER À CELLULES RÉNALES

(30) Priority: 02.09.2015 JP 2015173311
(43) Date of publication of application: 11.07.2018
(73) Proprietor: National Cancer Center, Tokyo 104-0045 (JP); Sekisui Medical Co., Ltd., Tokyo 103-0027 (JP)
(72) Inventor: KANAI, Yae, Tokyo 104-0045 (JP); ARAI, Eri, Tokyo 104-0045 (JP); NEMOTO, Yuriko, Tokyo 1030027 (JP); YOTANI, Takuya, Tokyo 1030027 (JP)
(74) Representative: Schiener, Jens
(86) International application number: PCT/JP2016/075842
(87) International publication number: WO 2017/038983

(56) References cited:
- EP-A1- 2 848 697
- WO-A1-2013/168644
- WO-A1-2014/136930
- WO-A1-2014/136930
- WO-A1-2015/129916
- YOTANI TAKUYA ET AL: "PROGNOSTICATION OF PATIENTS WITH CLEAR CELL CARCINOMAS BASED ON QUANTIFICATION OF DNA METHYLATION LEVELS OF CPG ISLAND METHYLATOR PHENOTYPE MARKER GENES USING ANION EXCHANGE HIGH PERFORMANCE LIQUID CHROMATOGRAPHY", CANCER RESEARCH, & 102ND ANNUAL MEETING OF THE AMERICAN-ASSOCIATION-FOR-CANCER-RESEARCH (AACR); ORLANDO, FL, USA; APRIL 02 -06, 2011, vol. 75, no. 15 SUPPL, 1 August 2015 (2015-08-01), page 1049, XP009508845, ISSN: 0008-5472, DOI: 10.1158/1538-7445.AM2015-1049
- E. ARAI ET AL: "Single-CpG-resolution methylome analysis identifies clinicopathologically aggressive CpG island methylator phenotype clear cell renal cell carcinomas", CARCINOGENESIS, vol. 33, no. 8, 18 May 2012 (2012-05-18), pages 1487-1493, XP055177040, ISSN: 0143-3334, DOI: 10.1093/carcin/bgs177
- YOTANI TAKUYA ET AL.: 'PROGNOSTICATION OF PATIENTS WITH CLEAR CELL CARCINOMAS BASED ON QUANTIFICATION OF DNA METHYLATION LEVELS OF CPG ISLAND METHYLATOR PHENOTYPE MARKER GENES USING ANION EXCHANGE HIGH PERFORMANCE LIQUID CHROMATOGRAPHY' CANCER RESEARCH vol. 75, no. SUPPL., page 1049, XP009508845

## Description

### Field of the Invention

The present invention relates to a method for determining the prognosis of renal cell carcinoma by use of the detection of methylated DNA.

### Background of the Invention

In recent years, abnormal methylation of DNA has been found to be deeply involved in malignant transformation and has received attention. Abnormal DNA methylation of CpG islands in some gene promoter regions is known as a characteristic epigenetic abnormality in cancer cells. The CpG island is a region in which a two-nucleotide sequence of cytosine (C)-guanine (G) via a phosphodiester bond (p) appears with high frequency. This region often resides in a promoter region upstream of a gene. The abnormal DNA methylation of the CpG island is involved in carcinogenesis through the inactivation of tumor suppressor genes, etc. DNA hypermethylation of the CpG island correlating with clinicopathological factors has been reported in colorectal cancer, stomach cancer, etc. (Non Patent Literatures 1 to 4). Such a type of cancer is called CpG island methylation phenotype (CIMP)-positive cancer.

Already established methods for analyzing methylated DNA include a method based on bisulfite reaction. This method is a method most generally used in the analysis of methylated DNA. The treatment of single-stranded DNA with bisulfite converts cytosine to uracil through sulfonation, hydrolic deamination, and desulfonation. On the other hand, methylated cytosine is left unaltered throughout the reaction time of actually performed bisulfite treatment because the reaction rate of sulfonation as the first step is very slow. Thus, PCR (polymerase chain reaction) using the bisulfite-treated DNA amplifies unmethylated cytosine with the uracil replaced with thymine, while leaving the methylated cytosine unaltered. The methylation status is analyzed through the use of the difference between the bases cytosine and thymine appearing in the sequence of this PCR amplification product. Methods generally used according to this basic principle are methylation-specific PCR (MSP) described in Patent Literature 1 and Non Patent Literature 5, and combined bisulfite restriction analysis (COBRA) described in Non Patent Literatures 6 and 7. The MSP method and the COBRA method are methylated DNA analysis methods currently used widely because these methods are capable of quantitatively analyzing methylated DNA without special equipment. A problem of the methods, however, is time and labor required for electrophoresis used in the analysis.

An alternative methylated DNA analysis method is pyrosequencing. This method involves subjecting a PCR amplification product of bisulfite-treated DNA to pyrosequencing, and detecting methylated cytosine replaced for thymine. However, the pyrosequencing requires a dedicated sequencer and also requires time-consuming analysis because the method reads bases one by one.

Recently, a method for determining a DNA methylation rate, comprising subjecting a PCR amplification product of bisulfite-treated DNA to ion exchange chromatography, and determining the DNA methylation rate on the basis of the retention time of a detection signal of the chromatography has been proposed (Patent Literature 2). This method has the advantage that the analysis time is drastically shortened as compared with the conventional methylated DNA analysis methods using electrophoresis or pyrosequencing.

Renal cell carcinoma (RCC) often develops even in middle-aged people belonging to the working population. A great majority of RCC case groups are completely cured by nephrectomy, whereas case groups which progress rapidly into distal metastasis are obviously present. These curable and metastatic RCC case groups largely differ in their clinical courses. Furthermore, some of cases with metastasis are known to respond to immunotherapy, molecular targeting therapeutic drugs, or the like. There is the possibility that the prognosis of cases likely to have recurrence can be improved by close follow-up, early diagnosis of recurrence, and additional aftercare. However, some cases experience rapid distal metastasis of clear cell RCC even having a low histopathological grade and a most common histological type. Thus, it is difficult to predict the prognosis of RCC using existing clinicopathological factors or the like.

Analyses by MSP, COBRA, and bacterial artificial chromosome (BAC) array-based methylated CpG island amplification (BAMCA) have showed that noncancerous renal cortical tissues obtained from RCC patients are already at a precancerous stage accompanied by change in DNA methylation status (Patent Literature 3 and Non Patent Literatures 8 to 11). In addition, genome-wide analysis by BAMCA has also revealed that change in DNA methylation in noncancerous renal cortical tissues at a precancerous stage is inherited to the corresponding RCC in the same patients. A method for predicting the prognosis of an RCC case has been successfully developed on the basis of this approach (Patent Literature 3 and Non Patent Literature 10).

Recently, it has been found that highly malignant RCC exhibits a CIMP-positive phenotype, and methylation at the CpG sites of 17 genes (FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2) is a feature of CIMP of RCC (Patent Literature 4). Patent Literature 4 has proposed a method for detecting a risk of poor prognosis of RCC by detecting a methylation level at the CpG sites of those 17 genes by bead array method, mass spectrometry (MassARRAY method), pyrosequencing, methylation-sensitive high-resolution melting curve analysis, quantitative PCR, direct sequencing of bisulfite treatment products, COBRA, or the like, and conducting clustering analysis based on the methylation level. Non Patent Literature 12 describes a determination of the prognosis of renal carcinoma patients based on a quantification of the methylation level of CpG islands and anion exchange chromatography.

### Citation List

### Patent Literature

[Patent Literature 1] U.S. Patent No. 5786146
[Patent Literature 2] WO 2014/136930
[Patent Literature 3] JP-A-2010-63413
[Patent Literature 4] WO 2013/168644

### [Non Patent Literature]

[Non Patent Literature 1] Nat. Rev. Cancer, 4, 988-993 (2004)
[Non Patent Literature 2] Proc. Natl. Acad. Sci. USA, 96, 8681-8686 (1999)
[Non Patent Literature 3] Proc. Natl. Acad. Sci. USA, 104, 18654-18659 (2007)
[Non Patent Literature 4] Cancer Res., 59, 5438-5442 (1999)
[Non Patent Literature 5] Proc. Natl. Acad. Sci. USA, 93, 9821-9826 (1996)
[Non Patent Literature 6] Nucleic Acids Res., 24, 5058-5059 (1996)
[Non Patent Literature 7] Nucleic Acids Res., 25, 2532-2534 (1997)
[Non Patent Literature 8] Clin. Cancer Res., 14, 5531-5539 (2008)
[Non Patent Literature 9] Int. J. Cancer, 119, 288-296 (2006)
[Non Patent Literature 10] Carcinogenesis, 30, 214-221 (2009)
[Non Patent Literature 11] Pathobiology, 78, 1-9 (2011)
[Non Patent Literature 12] Cancer Research, 75, Issue 15 Supplement, 1049, DOI: 10.1158/1538-7445

### Summary of the Invention

### Problem to be solved by the Invention

For preventing the recurrence of cancer, it is desirable to start treatment by obtaining information on the methylation status of genomic DNA and thereby determining a risk of recurrence with high specificity. If the metastasis and/or recurrence of cancer can be predicted and detected early, response thereof to immunotherapy, molecular targeting therapeutic drugs, or the like can be expected. Therefore, there has been a demand for a method capable of more precise prognosis of cancer. Furthermore, there has been a demand for a rapid and convenient method for precise prognosis of cancer.

### Means for solving the Invention

The present inventors have found that a signal obtained by subjecting a PCR amplification product of bisulfite-treated DNA to ion exchange chromatography differs between a CIMP-positive cancer group and a CIMP-negative cancer group, and the difference in the signal between the CIMP-positive cancer group and the CIMP-negative cancer group can be determined more accurately by analyzing a derivative value of the signal, consequently enabling more accurate determination of the prognosis of cancer.

Accordingly, the present invention provides the followings:
[1] A method for determining a tissue having renal cell carcinoma having poor prognosis, comprising:
   (1) subjecting sample DNA to ion exchange chromatography, wherein the sample DNA is obtained by treating target genomic DNA prepared from a renal tissue of a subject with bisulfite, followed by PCR amplification, wherein the subject is a renal cell carcinoma patient or a patient whose renal cell carcinoma has been treated by surgical operation, and who is in need of determination of the prognosis of the renal cell carcinoma;
   (2) calculating a derivative value of a detection signal of the chromatography; and
   (3) determining the renal tissue as being a tissue having renal cell carcinoma having poor prognosis when the derivative value calculated in the step (2) has two or more maximums;
   wherein the target genomic DNA comprises a CpG island in at least one gene selected from the group consisting of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2.
[2] A method for determining the prognosis of a renal cell carcinoma patient, comprising:
   (1) subjecting sample DNA to ion exchange chromatography, wherein the sample DNA is obtained by treating target genomic DNA prepared from a renal tissue of a subject with bisulfite, followed by PCR amplification, wherein the subject is a renal cell carcinoma patient or a patient whose renal cell carcinoma has been treated by surgical operation, and who is in need of determination of the prognosis of the renal cell carcinoma;
   (2) calculating a derivative value of a detection signal of the chromatography; and
   (3) determining the subject as being a patient with renal cell carcinoma having poor prognosis when the derivative value calculated in the step (2) has two or more maximums;
   wherein the target genomic DNA comprises a CpG island in at least one gene selected from the group consisting of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2.
[3] A method for obtaining data for determining a tissue having renal cell carcinoma, comprising:
   (1) subjecting sample DNA to ion exchange chromatography, wherein the sample DNA is obtained by treating target genomic DNA prepared from a renal tissue of a subject with bisulfite, followed by PCR amplification, wherein the subject is a renal cell carcinoma patient or a patient whose renal cell carcinoma has been treated by surgical operation, and who is in need of determination of the prognosis of the renal cell carcinoma;
   (2) calculating a derivative value of a detection signal of the chromatography; and
   (3) obtaining whether or not the derivative value calculated in the step (2) has two or more maximums as data for determining whether or not the tissue is a tissue having renal cell carcinoma having poor prognosis;
   wherein the target genomic DNA comprises a CpG island in at least one gene selected from the group consisting of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2.

### Effects of the Invention

The present invention provides a rapid, convenient, and highly accurate method for determining the prognosis of cancer. According to the present invention, a risk of recurrence in cancer patients can be determined more rapidly, conveniently, and highly accurately. Therefore, the present invention enables early resumption of treatment to cancer patients who are in need of treatment. Thus, the present invention contributes to improvement in the survival rate of cancer patients.

### Brief Description of the Drawings

[Figure 1] Figure 1 shows variation in chromatography retention time caused by DNA methylation rates. Figure 1A is chromatograms of DNAs differing in DNA methylation rate (0%, 25%, 50%, 75%, and 100%). Figure 1B is chromatograms of 50% methylated DNAs differing in DNA methylation position (random, closer to the 5' end, closer to the 3' end, and center).
[Figure 2] Figure 2 shows the correlation between DNA methylation rates and chromatography retention times.
[Figure 3] Figure 3 shows chromatograms of CIMP-positive specimen 01 (A) and CIMP-negative specimen 01 (B).
[Figure 4] Figure 4 shows chromatogram of CIMP-positive specimen 02.
[Figure 5] Figure 5 shows a chromatogram of CIMP-negative specimen 02.
[Figure 6] Figure 6 shows first derivative values of chromatography detection signals from CIMP-negative specimen 01, a negative control, and a positive control.
[Figure 7] Figure 7 shows first derivative values of chromatography detection signals from CIMP-positive specimen 01, a negative control, and a positive control.
[Figure 8] Figure 8 shows first derivative values of chromatography detection signals from CIMP-negative specimen 02, a negative control, and a positive control.
[Figure 9] Figure 9 shows first derivative values of chromatography detection signals from CIMP-positive specimen 02, a negative control, and a positive control.
[Figure 10] Figure 10 shows a chromatogram of CIMP-false negative specimen 01 with poor prognosis.
[Figure 11] Figure 11 shows a first derivative value of a chromatography detection signal from CIMP-false negative specimen 01.
[Figure 12] Figure 12 shows a chromatogram of CIMP-false negative specimen 02 with poor prognosis.
[Figure 13] Figure 13 shows a first derivative value of a chromatography detection signal from CIMP-false negative specimen 02.

### Embodiments for carrying out the Invention

In the present specification, the "renal cell carcinoma" is a cancer which develops by the malignant transformation of tubular epithelial cells of the kidney and is classified from its pathological features into clear cell type, granular cell type, chromophobe type, spindle type, cyst-associated type, type originating in cyst, cystic type, and papillary type.

In the present specification, examples of the "poor prognosis" of cancer include low prognostic (postoperative) survival rates of subjects and more specifically include a postoperative recurrence-free survival rate (tumor-free survival rate) of 50% or lower after a lapse of 500 days or a postoperative overall survival rate of 70% or lower after a lapse of 1,500 days.

In the present specification, the "DNA methylation" means a state where carbon at position 5 of cytosine in DNA is methylated. In the present specification, the phrase "detecting methylation" of DNA means to measure the presence or absence, abundance, or abundance ratio of methylated DNA in this DNA, or the methylation rate of this DNA. In the present specification, the "DNA methylation rate" means the proportion of methylated cytosine of a CpG island in particular DNA to be detected and can be indicated by, for example, the ratio of the number of methylated cytosine to the total number of cytosine (methylated cytosine and unmethylated cytosine) in the CpG island of the particular DNA to be detected.

In the present specification, the "CpG site" means a site where a phosphodiester bond (p) is formed between cytosine (C) and guanine (G) in DNA. In the present specification, the CpG island refers to a region in which a two-nucleotide sequence of cytosine (C)-guanine (G) via a phosphodiester bond (p) appears with high frequency. The CpG island often resides in a promoter region upstream of a gene. In the present specification, the "CpG site or CpG island of (a) gene" means a CpG island located at a position close to the coding region of the gene, or a CpG site contained in the CpG island, and preferably means a CpG site or a CpG island present in the promoter region of the gene. The CpG site or the CpG island of a particular gene can be identified on the basis of a method such as MassARRAY method or pyrosequencing.

In the present specification, the "retention time" means the time from analyte injection into a column through elution in chromatography such as column chromatography, and in other words, means the time during which the analyte is retained in the column. In the present specification, the "retention time serving as a reference" (hereinafter, also referred to as a reference retention time) refers to a HPLC retention time which can differentiate between a CIMP-positive group and a CIMP-negative group, or a HPLC retention time which can differentiate between a signal group derived from highly methylated DNA and a signal group derived from DNA having a low degree of methylation as to detection signals derived from genomic DNA prepared from renal cell carcinoma. Specifically, since a signal derived from highly methylated DNA is detected at a retention time shorter than the retention time serving as a reference (reference retention time), a specimen having a detection signal at a retention time shorter than the reference retention time can be determined as having poor prognosis. The reference retention time mentioned above can be set to a reference retention time appropriate for HPLC analysis conditions, a region of genomic DNA, or the type of a gene marker, etc., because a chromatogram varies depending on these conditions, etc. It is also preferred to set the reference retention time in consideration of necessary clinical sensitivity.

In the present specification, the "derivative value of a detection signal of the chromatography" is a value obtained by derivation of the detection signal (e.g., absorbance or fluorescence intensity) obtained in the chromatography with respect to the retention time. For example, a first derivative value obtained from a detection signal that exhibits a retention time having a single peak is typically represented by a curve having one maximum and one minimum.

In one embodiment, the present invention provides a method for determining a tissue having renal cell carcinoma, comprising:
(1) subjecting sample DNA to ion exchange chromatography, wherein the sample DNA is obtained by treating target genomic DNA prepared from a renal tissue of a subject with bisulfite, followed by PCR amplification;
(2) calculating a derivative value of a detection signal of the chromatography; and
(3) determining the renal tissue as being a tissue having renal cell carcinoma having poor prognosis when the derivative value calculated in the step (2) has two or more maximums, as further defined in claim 1.

In another embodiment, the present invention provides a method for obtaining data for determining a tissue having renal cell carcinoma, comprising:
(1) subjecting sample DNA to ion exchange chromatography, wherein the sample DNA is obtained by treating target genomic DNA prepared from a renal tissue of a subject with bisulfite, followed by PCR amplification;
(2) calculating a derivative value of a detection signal of the chromatography; and
(3) obtaining whether or not the derivative value calculated in the step (2) has two or more maximums as data for determining whether or not the tissue is a tissue having renal cell carcinoma having poor prognosis, as further defined in claim 3.

In an alternative embodiment, the present invention provides a method for determining the prognosis of a renal cell carcinoma patient, comprising:
(1) subjecting sample DNA to ion exchange chromatography, wherein the sample DNA is obtained by treating target genomic DNA prepared from a renal tissue of a subject with bisulfite, followed by PCR amplification;
(2) calculating a derivative value of a detection signal of the chromatography; and
(3) determining the subject as being a patient with renal cell carcinoma having poor prognosis when the derivative value calculated in the step (2) has two or more maximums, as further defined in claim 2.

In the method of the present invention, the subject is a renal cell carcinoma patient. Alternatively, the subject is a patient whose renal cell carcinoma has been treated by surgical operation, and who is in need of determination of the prognosis of the renal cell carcinoma.

The renal tissue of a subject can be a renal tissue containing genomic DNA or a cell thereof. Examples thereof include tissues collected by biopsy, surgical operation, or the like, and frozen products or fixed preparations thereof. It is desirable to use a frozen renal tissue from the viewpoint of suppressing the degradation, etc. of genomic DNA and more efficiently detecting DNA methylation.

The method for preparing genomic DNA from the renal tissue or the cell is not particularly limited, and an approach known in the art can be appropriately selected for use. Examples of the method known in the art for preparing DNA include phenol-chloroform method, and DNA extraction method as mentioned later using a commercially available DNA extraction kit, for example, QIAamp DNA Mini kit (manufactured by Qiagen N.V.), Clean Columns (manufactured by Hermes-NexTec GmbH), AquaPure (manufactured by Bio-Rad Laboratories, Inc.), ZR Plant/Seed DNA Kit (manufactured by Zymo Research Corp.), prepGEM (manufactured by ZyGEM NZ, Ltd.), or BuccalQuick (manufactured by TrimGen Corp.).

Subsequently, the extracted genomic DNA is treated with bisulfite. The method for treating the DNA with bisulfite is not particularly limited, and an approach known in the art can be appropriately selected for use. Examples of the method known in the art for bisulfite treatment include methods as mentioned above using a commercially available kit, for example, EpiTect Bisulfite Kit (48) (manufactured by Qiagen N.V.), MethylEasy (manufactured by Human Genetic Signatures Pty), Cells-to-CpG Bisulfite Conversion Kit (manufactured by Applied Biosystems, Inc.), or CpGenome Turbo Bisulfite Modification Kit (manufactured by Merck Millipore).

Subsequently, the bisulfite-treated genomic DNA is subjected to PCR to amplify the target genomic DNA. The PCR amplification method is not particularly limited, and an approach known in the art can be appropriately selected for use according to the sequence, length, amount, etc. of the target DNA to be amplified.

As for renal cell carcinoma, it has been reported that DNA methylation in 17 genes (FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2) is associated with the poor prognosis (CIMP-positive) of renal cell carcinoma (Patent Literature 4). Thus, in the method of the present invention, the target genomic DNA to be amplified by PCR is selected such that the DNA methylation of a CpG island in at least one gene selected from the group consisting of these 17 genes can be detected, and preferably selected such that the methylation of the CpG sites of these 17 genes can be detected. For example, the target genomic DNA is DNA encoding a portion or the whole of the coding region and/or the promoter region of any of the 17 genes. The target DNA is preferably DNA encoding a portion or the whole of the promoter region of any of the 17 genes, more preferably DNA encoding a portion or the whole of the CpG island of any of the 17 genes.

FAM150A is a gene encoding a protein specified by RefSeq ID: NP_997296; GRM6 is a gene encoding a protein specified by RefSeq ID: NP_000834; ZNF540 is a gene encoding a protein specified by RefSeq ID: NP_689819; ZFP42 is a gene encoding a protein specified by RefSeq ID: NP_777560; ZNF154 is a gene encoding a protein specified by RefSeq ID: NP_001078853; RIMS4 is a gene encoding a protein specified by RefSeq ID: NP_892015; PCDHAC1 is a gene encoding a protein specified by RefSeq ID: NP_061721; KHDRBS2 is a gene encoding a protein specified by RefSeq ID: NP_689901; ASCL2 is a gene encoding a protein specified by RefSeq ID: NP_005161; KCNQ1 is a gene encoding a protein specified by RefSeq ID: NP_000209; PRAC is a gene encoding a protein specified by RefSeq ID: NP_115767; WNT3A is a gene encoding a protein specified by RefSeq ID: NP_149122; TRH is a gene encoding a protein specified by RefSeq ID: NP_009048; FAM78A is a gene encoding a protein specified by RefSeq ID: NP_203745; ZNF671 is a gene encoding a protein specified by RefSeq ID: NP_079109; SLC13A5 is a gene encoding a protein specified by RefSeq ID: NP_808218; and NKX6-2 is a gene encoding a protein specified by RefSeq ID: NP_796374.

The CpG sites of the 17 genes are located at positions on the chromosomes described in Tables 1 to 4 on the basis of positions on the NCBI database Genome Build 37, which is a reference human genomic sequence.

**[Table 1]**

| Gene symbol | Chromosome number | Position on chromosome |
|---|---|---|
| FAM150A | 8 | 53478309 |
| | | 53478316,53478323 |
| | | 53478361, 53478363, 53478366 |
| | | 53478396, 53478403 |
| | | 53478426, 53478428 |
| | | 53478454 |
| | | 53478477 |
| | | 53478496, 53478499 |
| | | 53478504 |
| | | 53478511 |
| | | 53478536 |
| | | 53478585, 53478588, 53478592 |
| | | 53478624, 53478626 |
| GRM6 | 5 | 178422244 |
| | | 178422320, 178422324 |
| | | 178422375, 178422380 |
| ZNF540 | 19 | 38042472, 38042474 |
| | | 38042496 |
| | | 38042518 |
| | | 38042530, 38042532 |
| | | 38042544, 38042552 |
| | | 38042576 |
| | | 38042800, 38042802 |
| | | 38042816 |

**[Table 2]**

| Gene symbol | Chromosome number | Position on chromosome |
|---|---|---|
| ZFP42 | 4 | 188916867 |
| | | 188916875 |
| | | 188916899 |
| | | 188916913 |
| | | 188916982,188916984 |
| Z NF154 | 19 | 58220494 |
| | | 58220567 |
| | | 58220627 |
| | | 58220657, 58220662 |
| | | 58220706 |
| | | 58220768, 58220773 |
| RIMS4 | 20 | 43438576 |
| | | 43438621 |
| | | 43438865 |
| PCDHAC1 | 5 | 140306458 |
| KHDRBS2 | 6 | 62995963 |
| ASCL2 | 11 | 2292004 |
| | | 2292542, 2292544 |
| KCNQ1 | 11 | 2466409 |
| PRAC | 17 | 46799640 |
| | | 46799645, 46799648 |
| | | 46799654 |
| | | 46799745 |
| | | 46799755 |

**[Table 3]**

| Gene symbol | Chromosome number | Position on chromosome |
|---|---|---|
| WNT3A | 1 | 228194448 |
| | | 228195688 |
| | | 228195722 |
| | | 228195779 |
| TRH | 3 | 129693350, 129693352, 129693355, 129693358 |
| | | 129693406, 129693412 |
| | | 129693425 |
| | | 129693500 |
| | | 129693518, 129693521, 129693528 |
| | | 129693540, 129693543 |
| | | 129693563 |
| | | 129693570, 129693574 |
| | | 129693586 |
| | | 129693607 |
| | | 129693613 |
| | | 129693628 |
| | | 129693635 |
| | | 129693672 |
| FAM78A | 9 | 134152531 |
| ZNF671 | 19 | 58238740 |
| | | 58238780 |
| | | 58238810 |
| | | 58238850 |
| | | 58238928 |
| | | 58238954 |
| | | 58238987 |
| | | 58239012 |
| | | 58239027 |

**[Table 4]**

| Gene symbol | Chromosome number | Position on chromosome |
|---|---|---|
| SLC13A5 | 17 | 6616653, 6616655, 6616657 |
| | | 6616702, 6616705, 6616707 |
| | | 6616733 |
| | | 6616751 |
| | | 6616363, 6616768 |
| | | 6616812 |
| | | 6616826, 6616828 |
| | | 6616851, 6616854, 6616857 |
| | | 6616927, 6616929 |
| | | 6616968, 6616973 |
| | | 6617030, 6617038, 6617040, 6617044 |
| | | 6617077 |
| | | 6617124 |
| | | 6617251, 6617255 |
| | | 6617287, 6617291 |
| | | 6617300, 6617305 |
| | | 6617382 |
| | | 6617421, 6617423 |
| | | 6617456 |
| | | 6617466, 6617470 |
| | | 6617382 |
| | | 6617398, 6617402, 6617405 |
| | | 6617415 |
| | | 6617421, 6617423 |
| | | 6617466, 6617470 |
| | | 6617595, 6617597 |
| NKX6-2 | 10 | 134599860 |

The CpG site whose DNA methylation is to be detected is preferably at least one CpG site selected from the group consisting of chromosome 8 position 53,478,454, chromosome 5 position 178,422,244, chromosome 19 position 38,042,472, chromosome 4 position 188,916,867, chromosome 19 position 58,220,662, chromosome 20 position 43,438,865, chromosome 5 position 140,306,458, chromosome 6 position 62,995,963, chromosome 11 position 2,292,004, chromosome 11 position 2,466,409, chromosome 17 position 46,799,640, chromosome 19 position 58,220,494, chromosome 1 position 228,194,448, chromosome 3 position 129,693,613, chromosome 9 position 134,152,531, chromosome 19 position 58,238,928, chromosome 17 position 6,617,030, and chromosome 10 position 134,599,860 as positions on the NCBI database Genome Build 37, which is a reference human genomic sequence.

The chain length of the PCR amplification product can be appropriately selected in consideration of factors such as reduction in PCR amplification time and reduction in analysis time in ion exchange chromatography, and maintenance of separation performance. For example, the chain length of the PCR amplification product of target DNA rich in CpG islands is preferably 1,000 bp or shorter, more preferably 700 bp or shorter, further preferably 500 bp or shorter. On the other hand, the chain length of the PCR amplification product of target DNA having a few CpG islands is from 30 to 40 bp as the lower limit, which is the chain length of a PCR amplification product obtained using primers of approximately 15 mer in order to avoid nonspecific hybridization in PCR. Meanwhile, it is preferred to design primers such that the content of CpG islands is large. For example, cytosine of CpG sites is preferably contained at 2% or more, more preferably 5% or more, with respect to the chain length of the PCR amplification product.

Thus, preferred examples of the target genomic DNA to be amplified by PCR in the method of the present invention include:
DNA comprising the CpG island of SLC13A5 which is amplified by a primer set represented by SEQ ID NOs: 17 and 18, SEQ ID NOs: 19 and 20, or SEQ ID NOs: 21 and 22;
DNA comprising the CpG island of FAM150A which is amplified by a primer set represented by SEQ ID NOs: 23 and 24 or SEQ ID NOs: 51 and 52;
DNA comprising the CpG island of GRM6 which is amplified by a primer set represented by SEQ ID NOs: 25 and 26;
DNA comprising the CpG island of ZFP42 which is amplified by a primer set represented by SEQ ID NOs: 27 and 28;
DNA comprising the CpG island of ZNF154 which is amplified by a primer set represented by SEQ ID NOs: 29 and 30;
DNA comprising the CpG island of RIMS4 which is amplified by a primer set represented by SEQ ID NOs: 31 and 32;
DNA comprising the CpG island of TRH which is amplified by a primer set represented by SEQ ID NOs: 33 and 34;
DNA comprising the CpG island of ZNF540 which is amplified by a primer set represented by SEQ ID NOs: 35 and 36;
DNA comprising the CpG island of PCDHAC1 which is amplified by a primer set represented by SEQ ID NOs: 37 and 38;
DNA comprising the CpG island of PRAC which is amplified by a primer set represented by SEQ ID NOs: 39 and 40;
DNA comprising the CpG island of ZNF671 which is amplified by a primer set represented by SEQ ID NOs: 41 and 42;
DNA comprising the CpG island of WNT3A which is amplified by a primer set represented by SEQ ID NOs: 43 and 44;
DNA comprising the CpG island of KHDRBS2 which is amplified by a primer set represented by SEQ ID NOs: 45 and 46; and
DNA comprising the CpG island of ASCL2 which is amplified by a primer set represented by SEQ ID NOs: 47 and 48.

Alternatively, preferred examples of the target genomic DNA to be amplified by PCR in the method of the present invention include DNA comprising the CpG island of FAM150A represented by SEQ ID NO: 50.

Subsequently, the obtained PCR amplification product is subjected as sample DNA to ion exchange chromatography. The ion exchange chromatography according to the present invention is preferably anion exchange chromatography. The column packing material for use in the ion exchange chromatography according to the present invention is not particularly limited as long as the packing material is substrate particles having a strong cationic group on the surface. Substrate particles having both a strong cationic group and a weak cationic group on the surface of the packing material as shown in WO 2012/108516 are preferred.

In the present specification, the strong cationic group means a cationic group which is dissociated in a wide pH range of from 1 to 14. Specifically, the strong cationic group can maintain its dissociated (cationized) state without being influenced by the pH of an aqueous solution.

Examples of the strong cationic group include quaternary ammonium groups. Specific examples thereof include trialkylammonium groups such as a trimethylammonium group, a triethylammonium group, and a dimethylethylammonium group. Examples of the counter ion for the strong cationic group include halide ions such as a chloride ion, a bromide ion, and an iodide ion.

The amount of the strong cationic group introduced to the surface of the substrate particles is not particularly limited and is preferably 1 µeq/g as the lower limit and 500 µeq/g as the upper limit with respect to the dry weight of the packing material. If the amount of the strong cationic group is less than 1 µeq/g, separation performance may be deteriorated due to weak retention strength. If the amount of the strong cationic group exceeds 500 µeq/g, retention strength may be too strong to easily elute the sample DNA, resulting in problems such as too long an analysis time.

In the present specification, the weak cationic group means a cationic group having pka of 8 or higher. Specifically, the weak cationic group changes its dissociated state by the influence of the pH of an aqueous solution. Specifically, at pH higher than 8, the proton of the weak cationic group is dissociated so that the ratio of a group having no positive charge is increased. On the other hand, at pH lower than 8, the weak cationic group is protonated so that the ratio of a group having positive charge is increased.

Examples of the weak cationic group include tertiary amino groups, secondary amino groups, and primary amino groups. Among them, a tertiary amino group is desirable.

The amount of the weak cationic group introduced to the surface of the substrate particles is not particularly limited and is preferably 0.5 µeq/g as the lower limit and 500 µeq/g as the upper limit with respect to the dry weight of the packing material. If the amount of the weak cationic group is less than 0.5 µeq/g, separation performance may not be improved due to too small an amount. If the amount of the weak cationic group exceeds 500 µeq/g, retention strength may be too strong to easily elute the sample DNA, resulting in problems such as too long an analysis time, as with the strong cationic group.

The amount of the strong cationic group or the weak cationic group on the surface of the substrate particles can be measured by quantifying a nitrogen atom contained in an amino group. Examples of the method for quantifying nitrogen include Kjeldahl method. In the case of the packing material described in the present invention (Examples), first, nitrogen contained in the strong cationic group after polymerization is quantified. Subsequently, nitrogen contained in the strong cationic group and the weak cationic group after introduction of the weak cationic group is quantified. As a result, the amount of the weak cationic group introduced later can be calculated. Such quantification allows the amount of the strong cationic group and the amount of the weak cationic group to be adjusted within the ranges described above for preparing the packing material.

For example, synthetic polymer fine particles obtained using polymerizable monomers or the like, or inorganic fine particles such as fine silica particles can be used as the substrate particles. Hydrophobic cross-linked polymer particles consisting of a synthetic organic polymer are desirable.

The hydrophobic cross-linked polymer may be any of a hydrophobic cross-linked polymer obtained by copolymerizing at least one hydrophobic cross-linkable monomer and at least one monomer having a reactive functional group, and a hydrophobic cross-linked polymer obtained by copolymerizing at least one hydrophobic cross-linkable monomer, at least one monomer having a reactive functional group, and at least one hydrophobic non-cross-linkable monomer.

The hydrophobic cross-linkable monomer is not particularly limited as long as the monomer has two or more vinyl groups in one molecule. Examples thereof include: di(meth)acrylic acid esters such as ethylene glycol di(meth)acrylate, polyethylene glycol di(meth)acrylate, propylene glycol di(meth)acrylate, and polypropylene glycol di(meth)acrylate; tri(meth)acrylic acid esters such as trimethylol methane tri(meth)acrylate and tetramethylol methane tri(meth)acrylate; tetra(meth)acrylic acid esters; and aromatic compounds such as divinylbenzene, divinyltoluene, divinylxylene, and divinylnaphthalene. In the present specification, the (meth)acrylate means acrylate or methacrylate, and (meth)acryl means acryl or methacryl.

Examples of the monomer having a reactive functional group include glycidyl (meth)acrylate and isocyanatoethyl (meth)acrylate.

The hydrophobic non-cross-linkable monomer is not particularly limited as long as the monomer is a non-cross-linkable polymerizable organic monomer having hydrophobic properties. Examples thereof include: (meth)acrylic acid esters such as methyl (meth)acrylate, ethyl (meth)acrylate, butyl (meth)acrylate, and t-butyl (meth)acrylate; and styrene monomers such as styrene and methylstyrene.

When the hydrophobic cross-linked polymer is obtained by copolymerizing the hydrophobic cross-linkable monomer and the monomer having a reactive functional group, the content ratio of a segment derived from the hydrophobic cross-linkable monomer in the hydrophobic cross-linked polymer is preferably 10 wt% as the lower limit, more preferably 20 wt% as the lower limit.

The packing material for the ion exchange chromatography used in the present invention preferably has a polymer layer having the strong cationic group and the weak cationic group on the surface of the substrate particles. For the polymer having the strong cationic group and the weak cationic group, it is preferred that the strong cationic group and the weak cationic group should be respectively derived from independent monomers. Specifically, the packing material for the ion exchange chromatography used in the present invention is preferably a packing material in which the weak cationic group is introduced in the surface of coated polymer particles consisting of the hydrophobic cross-linked polymer particles and a layer of a hydrophilic polymer having the strong cationic group copolymerized at the surface of the hydrophobic cross-linked polymer particles.

The hydrophilic polymer having the strong cationic group is formed from hydrophilic monomers having the strong cationic group and can contain a segment derived from one or more hydrophilic monomers having the strong cationic group. Specifically, examples of the method for producing the hydrophilic polymer having the strong cationic group include a method which involves homopolymerizing a hydrophilic monomer having the strong cationic group, a method which involves copolymerizing two or more hydrophilic monomers each having the strong cationic group, and a method which involves copolymerizing a hydrophilic monomer having the strong cationic group and a hydrophilic monomer having no strong cationic group.

The hydrophilic monomer having the strong cationic group preferably has a quaternary ammonium group. Specific examples thereof include ethyl methacrylate triethylammonium chloride, ethyl methacrylate dimethylethylammonium chloride, ethyl methacrylate dimethylbenzylammonium chloride, ethyl acrylate dimethylbenzylammonium chloride, ethyl acrylate triethylammonium chloride, ethyl acrylate dimethylethylammonium chloride, acrylamide ethyltrimethylammonium chloride, acrylamide ethyltriethylammonium chloride, and acrylamide ethyl dimethylethylammonium chloride.

A method known in the art can be used as a method for introducing the weak cationic group to the surface of the coated polymer particles. Specifically, examples of the method for introducing a tertiary amino group as the weak cationic group include: a method which involves copolymerizing the hydrophilic monomer having the strong cationic group at the surface of the hydrophobic cross-linked polymer particles consisting of a hydrophobic cross-linked polymer having a segment derived from a monomer having a glycidyl group, and subsequently reacting the glycidyl group with a reagent having a tertiary amino group; a method which involves copolymerizing the hydrophilic monomer having the strong cationic group at the surface of the hydrophobic cross-linked polymer particles consisting of a hydrophobic cross-linked polymer having a segment derived from a monomer having an isocyanate group, and subsequently reacting the isocyanate group with a reagent having a tertiary amino group; a method which involves copolymerizing the hydrophilic monomer having the strong cationic group and a monomer having a tertiary amino group at the surface of the hydrophobic cross-linked polymer particles; a method which involves introducing a tertiary amino group to the surface of the coated polymer particles having a hydrophilic polymer layer having the strong cationic group using a silane coupling agent having the tertiary amino group; a method which involves copolymerizing the hydrophilic monomer having the strong cationic group at the surface of the hydrophobic cross-linked polymer particles consisting of a hydrophobic cross-linked polymer having a segment derived from a monomer having a carboxy group, and subsequently condensing the carboxy group with a reagent having a tertiary amino group using carbodiimide; and a method which involves copolymerizing the hydrophilic monomer having the strong cationic group at the surface of the hydrophobic cross-linked polymer particles consisting of a hydrophobic cross-linked polymer having a segment derived from a monomer having an ester bond, hydrolyzing the ester bond moiety, and then condensing a carboxy group formed by the hydrolysis with a reagent having a tertiary amino group using carbodiimide. Among them, the method which involves copolymerizing the hydrophilic monomer having the strong cationic group at the surface of the hydrophobic cross-linked polymer particles consisting of a hydrophobic cross-linked polymer having a segment derived from a monomer having a glycidyl group, and subsequently reacting the glycidyl group with a reagent having a tertiary amino group, or the method which involves copolymerizing the hydrophilic monomer having the strong cationic group at the surface of the hydrophobic cross-linked polymer particles consisting of a hydrophobic cross-linked polymer having a segment derived from a monomer having an isocyanate group, and subsequently reacting the isocyanate group with a reagent having a tertiary amino group, is preferred.

The reagent having a tertiary amino group which is reacted with the reactive functional group such as a glycidyl group or an isocyanate group is not particularly limited as long as the reagent has a functional group reactable with the tertiary amino group and the reactive functional group. Examples of the functional group reactable with the tertiary amino group and the reactive functional group include primary amino groups and a hydroxy group. Among others, a group having a terminal primary amino group is preferred. Specific examples of the reagent having the functional group include N,N-dimethylaminomethylamine, N,N-dimethylaminoethylamine, N,N-dimethylaminopropylamine, N,N-dimethylaminobutylamine, N,N-diethylaminoethylamine, N,N-diethylaminopropylethylamine, N,N-diethylaminobutylamine, N,N-diethylaminopentylamine, N,N-diethylaminohexylamine, N,N-dipropylaminobutylamine, and N,N-dibutylaminopropylamine.

For the relative positional relationship between the strong cationic group (preferably, a quaternary ammonium salt) and the weak cationic group (preferably, a tertiary amino group), it is preferred that the strong cationic group should be positioned more distant than the weak cationic group from the surface of the substrate particles, i.e., positioned on the outer side of the weak cationic group. Preferably, for example, the weak cationic group is located within 30 angstroms from the surface of the substrate particles, and the strong cationic group is located within 300 angstroms from the surface of the substrate particles and on the outer side of the weak cationic group.

The average particle size of the substrate particles which are used as the packing material for the ion exchange chromatography used in the present invention is not particularly limited and is preferably 0.1 µm as the lower limit and 20 µm as the upper limit. If the average particle size is less than 0.1 µm, poor separation may occur due to too high an intra-column pressure. If the average particle size exceeds 20 µm, poor separation may occur due to too large an intra-column dead volume. In the present specification, the average particle size refers to a volume-average particle size and can be measured using a particle size distribution measurement apparatus (e.g., AccuSizer 780, manufactured by Particle Sizing Systems).

Conditions known in the art can be used for the composition of an eluent for use in the ion exchange chromatography according to the present invention.

The buffer solution for use in the eluent is preferably a buffer solution containing a salt compound known in the art, or an organic solvent. Specific examples thereof include a tris-HCl buffer solution, a TE buffer solution consisting of tris and EDTA, and a TBA buffer solution consisting of tris, boric acid, and EDTA.

The pH of the eluent is not particularly limited and is preferably 5 as the lower limit and 10 as the upper limit. At the pH set to within this range, the weak cationic group is considered to also work effectively as an ion exchange group (anion exchange group). The pH of the eluent is more preferably 6 as the lower limit and 9 as the upper limit.

Examples of the salt contained in the eluent include: salts consisting of a halide and an alkali metal, such as sodium chloride, potassium chloride, sodium bromide, and potassium bromide; and salts consisting of a halide and an alkaline earth metal, such as calcium chloride, calcium bromide, magnesium chloride, and magnesium bromide; and inorganic acid salts such as sodium perchlorate, potassium perchlorate, sodium sulfate, potassium sulfate, ammonium sulfate, sodium nitrate, and potassium nitrate. Alternatively, an organic acid salt such as sodium acetate, potassium acetate, sodium succinate, or potassium succinate may be used. Any one of these salts may be used alone or, two or more thereof may be used in combination.

The salt concentration of the eluent can be appropriately adjusted according to analysis conditions and is preferably 10 mmol/L as the lower limit and 2,000 mmol/L as the upper limit, more preferably 100 mmol/L as the lower limit and 1,500 mmol/L as the upper limit.

The eluent for use in the ion exchange chromatography used in the present invention further contains an anti-chaotropic ion for further enhancing separation performance. The anti-chaotropic ion has properties opposite to those of a chaotropic ion and works to stabilize a hydrated structure. Therefore, the anti-chaotropic ion is effective for strengthening the hydrophobic interaction between the packing material and a nucleic acid molecule. The main interaction of the ion exchange chromatography used in the present invention is electrostatic interaction. Separation performance is enhanced through the use of the work of the hydrophobic interaction in addition thereto.

Examples of the anti-chaotropic ion contained in the eluent include a phosphate ion (PO₄³⁻) , a sulfate ion (SO₄²⁻) , an ammonium ion (NH₄⁺) , a potassium ion (K⁺), and a sodium ion (Na⁺). Among combinations of these ions, a sulfate ion and an ammonium ion are preferably used. Any one of these anti-chaotropic ions may be used alone, or two or more thereof may be used in combination. Some of the anti-chaotropic ions mentioned above comprise a salt contained in the eluent or a component of the buffer solution. Use of such a component is suitable for the present invention, because the component possesses both of properties or buffering ability as the salt contained in the eluent and properties as the anti-chaotropic ion.

The concentration at the time of analysis of the anti-chaotropic ion in the eluent for the ion exchange chromatography used in the present invention can be appropriately adjusted according to an analyte and is desirably 2,000 mmol/L or lower in terms of anti-chaotropic salt. Specific examples of such a method can include a method which involves performing gradient elution at anti-chaotropic salt concentrations ranging from 0 to 2,000 mmol/L. Thus, the concentration of the anti-chaotropic salt at the start of analysis does not have to be 0 mmol/L, and the concentration of the anti-chaotropic salt at the completion of analysis does not have to be 2,000 mmol/L. The gradient elution method may be a low-pressure gradient method or may be a high-pressure gradient method. The method preferably involves performing elution while the concentration is precisely adjusted by the high-pressure gradient method.

The anti-chaotropic ion may be added to only one eluent for use in elution or may be added to a plurality of eluents. Also, the anti-chaotropic ion may play a role both in the effect of enhancing the hydrophobic interaction between the packing material and the sample DNA or the buffering ability and in the effect of eluting the sample DNA from the column.

The column temperature for analyzing the sample DNA by the ion exchange chromatography according to the present invention is preferably 30°C or higher, more preferably 40°C or higher, further preferably 45°C or higher. If the column temperature in the ion exchange chromatography is lower than 30°C, the hydrophobic interaction between the packing material and the sample DNA is weakened, and the desired separating effect is difficult to obtain. If the column temperature in the ion exchange chromatography is lower than 45°C, the PCR amplification product of bisulfite-treated methylated DNA (methylated DNA sample) and the PCR amplification product of bisulfite-treated unmethylated DNA (unmethylated DNA sample) do not much differ in retention time. When the column temperature is 60°C or higher, the methylated DNA sample and the unmethylated DNA sample differ more largely in retention time and respectively exhibit more clear peaks. Therefore, DNA methylation can be detected more accurately.

As the column temperature in the ion exchange chromatography is higher, the methylated DNA sample and the unmethylated DNA sample are more clearly separable. Therefore, the methylated DNA and the unmethylated DNA tend to differ in their peak areas or peak heights at retention times according to their abundance ratios in the target DNA. Thus, at a higher column temperature, the respective abundances or abundance ratios of the methylated DNA and the unmethylated DNA in the target DNA can be measured more easily on the basis of the difference between the peak areas or heights at retention times of the methylated DNA sample and the unmethylated DNA sample.

On the other hand, a column temperature of 90°C or higher in the ion exchange chromatography is not preferred for the analysis because two strands of the nucleic acid molecule in the sample DNA are dissociated. A column temperature of 100°C or higher is not preferred for the analysis because the eluent might be boiled. Thus, the column temperature for analyzing the sample DNA by the ion exchange chromatography according to the present invention can be 30°C or higher and lower than 90°C and is preferably 40°C or higher and lower than 90°C, more preferably 45°C or higher and lower than 90°C, further preferably 55°C or higher and lower than 90°C, still further preferably 55°C or higher and 85°C or lower, particularly preferably 60°C or higher and 85°C or lower.

The sample injection volume to the ion exchange chromatography column is not particularly limited and can be appropriately adjusted according to the ion exchange capacity of the column and the sample concentration. The flow rate is preferably from 0.1 mL/min to 3.0 mL/min, more preferably from 0.5 mL/min to 1.5 mL/min. At a slower flow rate, improved separation can be expected. Too slow a flow rate might require a long time for analysis or incur reduction in separation performance due to broader peaks. On the other hand, a faster flow rate is advantageous in terms of reduction in analysis time, but incurs reduction in separation performance due to peak compression. Accordingly, it is desirable to set the flow rate to within the range described above, though this parameter is appropriately adjusted according to the performance of the column. The retention time of each sample can be predetermined by a preliminary experiment on each sample. A flowing method known in the art, such as linear gradient elution method or stepwise elution method can be used. The flowing method according to the present invention is preferably linear gradient elution method. The amplitude of the gradient can be appropriately adjusted within a range of the eluent for use in elution from 0% to 100% according to the separation performance of the column and the characteristics of the analyte (here, the sample DNA).

In the present invention, the PCR amplification product of the bisulfite-treated target DNA (i.e., sample DNA) is subjected to ion exchange chromatography by the procedures described above.

The treatment of DNA with bisulfite converts unmethylated cytosine in the DNA to uracil, while leaving methylated cytosine unaltered. The PCR amplification of the bisulfite-treated DNA further replaces uracil derived from the unmethylated cytosine with thymine and therefore results in the difference in the abundance ratios of cytosine and thymine between methylated DNA and unmethylated DNA. Thus, the sample DNA has a distinctive sequence according to the methylation rate of the original target genomic DNA. The sample DNA is subjected to ion exchange chromatography to obtain a chromatogram showing a distinctive signal according to its nucleotide sequence. Thus, the methylation of the target genomic DNA can be detected on the basis of a detection signal obtained by the ion exchange chromatography of the sample DNA.

The presence or absence of methylated DNA in sample DNA can be measured, for example, by comparing a detection signal from the PCR amplification product of the bisulfite-treated target DNA (i.e., sample DNA) with a detection signal from the PCR amplification product of bisulfite-treated DNA having the same nucleotide sequence, albeit not methylated, as that of the target DNA (hereinafter, this PCR amplification product is referred to as a negative control), or a detection signal from the PCR amplification product of bisulfite-treated DNA having the same nucleotide sequence as that of the target DNA and having a known methylation rate (e.g., 100%) (hereinafter, this PCR amplification product is referred to as a positive control).

Alternatively, the ratio between the abundance of methylated DNA and the abundance of unmethylated DNA in target DNA can be measured by comparing a detection signal from the sample DNA with detection signals from the negative and positive controls. Alternatively, the methylation rate of methylated DNA, its abundance, and the ratio between the abundance of methylated DNA and the abundance of unmethylated DNA in target DNA can be measured by comparing detection signals from a plurality of PCR amplification products derived from a plurality of bisulfite-treated DNAs each having the same nucleotide sequence as that of the target DNA and having a known methylation rate (hereinafter, these PCR amplification products are referred to as standards) with a detection signal from the sample DNA.

Thus, the methylation of the sample DNA can be detected by comparing a detection signal from the sample DNA obtained in the chromatography with a detection signal from the negative or positive control, or the standards, on the basis of difference between their detection signals.

DNA synthesized chemically or in a genetic engineering manner may be used as the DNA of the negative control, the positive control, or the standards. A commercially available product can also be used in the preparation of the negative control, the positive control, and the standards, and, for example, EpiTect Control DNA and Control DNA Set (manufactured by Qiagen N.V.) can be used.

For example, in the ion exchange chromatography, the sample DNA and the negative control, the positive control, or the standards can be individually subjected to ion exchange chromatography analysis. The samples adsorbed on the column can be applied to gradient elution using a plurality of eluents to elute the sample DNA and the negative control, the positive control, or the standards at different retention times according to their DNA methylation rates.

The detection signal from the negative control can be acquired by performing bisulfite treatment and PCR according to the procedures mentioned above using DNA having the same nucleotide sequence, albeit not methylated, as that of the target DNA instead of the sample DNA and subjecting the obtained PCR amplification product to ion exchange chromatography. The detection signal from the positive control can be acquired by performing bisulfite treatment and PCR according to the procedures mentioned above using DNA having the same nucleotide sequence as that of the target DNA and having a known methylation rate (e.g., 100%) instead of the sample DNA and subjecting the obtained PCR amplification product to ion exchange chromatography. Alternatively, the detection signal from the negative or positive control may be obtained by subjecting the synthesized DNA or the commercially available DNA mentioned above as the negative or positive control to ion exchange chromatography.

For example, the target DNA can be determined as methylated when the peak retention time of the detection signal obtained from the sample DNA deviates from the peak retention time of the negative control. In this respect, as the deviation of the retention time is larger, the methylation rate can be presumed to be larger. On the other hand, as the peak retention time of the detection signal obtained from the sample DNA deviates more largely from the peak retention time of the 100% methylated positive control, the methylation rate of the target DNA can be presumed to be smaller.

The detection signals from the standards can be acquired by performing bisulfite treatment and PCR according to the procedures mentioned above using a plurality of DNAs each having the same nucleotide sequence as that of the target DNA and having a known methylation rate instead of the sample DNA and subjecting each of a plurality of the obtained PCR amplification products to ion exchange chromatography. Furthermore, a calibration curve may be prepared from the respective detection signals thus obtained. Alternatively, the detection signals from the standards may be obtained by subjecting the synthesized DNA or the commercially available DNA mentioned above as the standards to ion exchange chromatography.

The calibration curve can establish an association between DNA methylation rates and retention times. Thus, a DNA methylation rate corresponding to the reference retention time (hereinafter, also referred to as a reference DNA methylation rate) can be determined on the basis of the calibration curve. Provided that the reference DNA methylation rate is obtained in advance, a new reference retention time can be easily calculated, even if HPLC equipment or analysis conditions are changed, by applying the reference DNA methylation rate to a calibration curve newly prepared using the changed equipment or conditions.

Also, the abundance ratio of methylated DNA (e.g., the abundance ratio of unmethylated DNA or the abundance ratio of DNA methylated at a particular rate) in target DNA can be determined, for example, by comparing the peak height or the peak area of the detection signal obtained from the sample DNA with the peak height or the peak area of a detection signal obtained from the PCR amplification product of bisulfite-treated DNA having a known methylation rate and mixing ratio of methylated DNA.

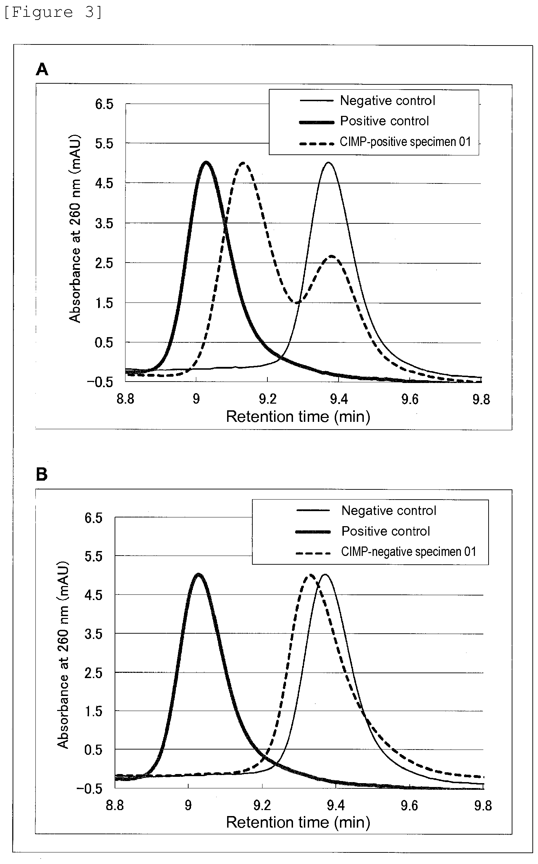

The prognosis of renal cell carcinoma in the subject can be determined on the basis of the retention time of the detection signal obtained by the chromatography. Specifically, the target DNA has a high methylation rate when the obtained detection signal has a peak at a short retention time as shown in Figure 3A as a result of the chromatography of the sample DNA. This indicates that the target DNA is derived from a tissue having CIMP-positive renal cell carcinoma having poor prognosis. On the other hand, the target DNA has a low methylation rate when the obtained detection signal has a peak at a long retention time as shown in Figure 3B as a result of the chromatography of the sample DNA. This indicates that the target DNA is derived from a tissue having CIMP-negative renal cell carcinoma.

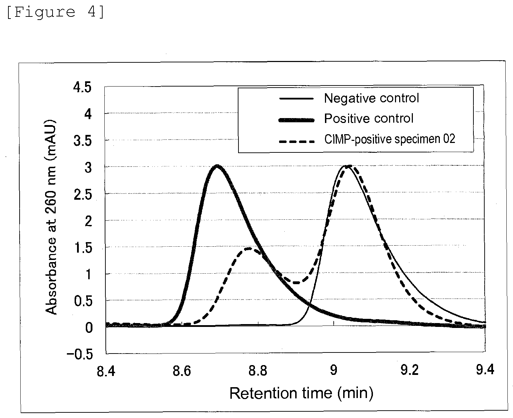

For example, the target DNA is determined as a DNA obtained from a renal cell carcinoma patient with poor prognosis when the detection signal is obtained at a retention time shorter than the reference retention time. As shown in Figure 3A, for example, the peak of DNA having a high methylation rate and the peak of DNA having a low methylation rate are easily separable. In the case of bimodal peaks, the influence of unmethylated DNA can be removed by the separation between the peaks, and the DNA obtained from a renal cell carcinoma patient with poor prognosis can be determined accurately. As shown in Figure 4, even when the peak of DNA having a low methylation rate is higher than the peak of DNA having a high methylation rate, the DNA obtained from a renal cell carcinoma patient with poor prognosis can be determined accurately.

Furthermore, difference data can be determined by subtracting the detection signal obtained from the negative control from the detection signal obtained from the sample DNA. By determining the difference data, a signal from unmethylated DNA (noise) can be removed from detection signals as the whole sample DNA to extract only a signal from methylated DNA. The difference data corresponds to a detection signal derived from methylated DNA in the target DNA. The retention time of this difference data is compared with the reference retention time. The target DNA is determined as a DNA obtained from a renal cell carcinoma patient with poor prognosis when the result is shorter than the reference retention time. Use of the difference data permits detection or analysis of methylated DNA even in a sample in which a signal component from the methylated DNA is detected only at a weak level, for example, DNA having a low abundance ratio of the methylated DNA or DNA containing methylated DNA having a low methylation rate. When the target DNA contains DNAs having various methylation rates, various chromatogram patterns having, for example, a shoulder peak or overlapping peaks, are obtained. In such a case, the prognosis of cancer can be determined highly accurately by determining the difference data, because only a signal of DNA having a high methylation rate in the DNA can be extracted.

Thus, use of the difference data permits more highly accurate analysis on sample DNA. For determining the difference data, it is desirable to use equal DNA levels of the sample DNA and the DNA used as the negative control. The DNA levels can be confirmed by a measurement method such as absorbance measurement.

The procedures of the method for determining the prognosis of cancer according to the present invention using the difference data are basically the same as those for obtaining the data before the subtraction as mentioned above. For example, the retention time of the detection signal obtained by the chromatography is examined, and as a result, the target DNA is determined as a DNA obtained from a renal cell carcinoma patient with poor prognosis when the detection signal is obtained at a retention time shorter than the retention time serving as a reference.

In the prognosis of renal cell carcinoma, use of the difference data is very effective. A specimen which is collected for clinical examination may contain normal cells such as noncancerous epithelial cells or stromal cells in which DNA methylation has not yet proceeded, or may be rich in cells in a precancerous state in which DNA methylation has not much proceeded. Alternatively, such a specimen may have various ratios of cancer cells having various DNA methylation rates. Use of the difference data can remove the influence of normal cells in the specimen, normal DNA in precancerous cells, or unmethylated DNA derived from cancer cells in which a gene region to be assayed is not methylated. Therefore, the methylated DNA can be detected more highly accurately. Furthermore, the prognosis of cancer can be determined more highly accurately by use of the detection result.

According to the method, methylated DNA in a sample can be separated from unmethylated DNA and detected. Therefore, even if the sample is rich in normal cells, the presence of methylated DNA and the methylation rate thereof can be detected highly accurately to precisely determine the prognosis. The method of the present invention permits precise determination of prognosis even for a subject which has not been determined as CIMP-positive by conventional examination in spite of poor prognosis.

Examples of the method for determining the presence or absence of the peak of the detection signal obtained by the chromatography include peak detection using existing data processing software, for example, LCsolution (Shimadzu Corp.), GRAMS/AI (Thermo Fisher Scientific, Inc.), or Igor Pro (WaveMetrics). The method for determining the presence or absence of the peak using LCsolution will be described as an example. Specifically, a retention time zone in which a peak is to be detected is first designated. Next, various parameters are set in order to remove unnecessary peaks such as noise. Examples of such settings include setting of the parameter "WIDTH" to larger than the half widths of unnecessary peaks, setting of the parameter "SLOPE" to larger than the leading slopes of unnecessary peaks, and changing of the parameter "DRIFT" setting to select either vertical partitioning or baseline partitioning of peaks with a low degree of separation. The values of these parameters can be set to appropriate values according to a chromatogram because the obtained chromatogram differs depending on analysis conditions, the type of a selected gene marker, the amount of a specimen, etc.

The retention time, i.e., peak top time, can be automatically calculated using the data processing software. For example, first derivation of the chromatogram is carried out, and the time at which the derivative changes from positive to negative can be obtained as the peak top time.

In the method of the present invention, a derivative value of the detection signal of the chromatography obtained from the sample DNA is further calculated. The derivative value of the detection signal of the chromatography can be automatically calculated using the data processing software mentioned above. Alternatively, the derivative value can be calculated using spreadsheet software (Microsoft(R) Excel(R), etc.). Even when the retention time of the detection signal of the chromatography is obscure, for example, even when the detection signal has a plurality of overlapping peaks or has a shoulder peak, a sample derived from a renal cell carcinoma patient with poor prognosis and a sample derived from a renal cell carcinoma patient with good prognosis can be distinguished therebetween by determining the derivative value. More specifically, a first derivative value (i.e., slope) of the detection signal of the chromatography obtained from the sample DNA is plotted against the retention time. In this case, the target DNA is determined as being DNA derived from a tissue having renal cell carcinoma having good prognosis when a curve having one maximum is drawn. In addition, a subject which has provided the target DNA is determined as being a patient with renal cell carcinoma having good prognosis. On the other hand, a first derivative value of the detection signal of the chromatography obtained from the sample DNA is plotted against the retention time as described above. In this case, the target DNA is determined as being DNA derived from a tissue having renal cell carcinoma having poor prognosis when a curve having two or more maximums is drawn. In addition, a subject which has provided the target DNA is determined as being a patient with renal cell carcinoma having poor prognosis. For example, in one aspect of the method of the present invention, sample DNA whose first derivative value is represented by a curve having two or more maximums is selected, and this is DNA derived from a tissue having renal cell carcinoma having poor prognosis, or DNA derived from a renal cell carcinoma patient with poor prognosis.

The time range in which the derivative value of the detection signal from the sample DNA is calculated can be appropriately set and can be set to, for example, the range from the peak top retention time of the detection signal from the positive control to the peak top retention time of the detection signal from the negative control. Alternatively, the time range in which the derivative value is calculated may be set in consideration of assay accuracy and may be set to, for example, a range that attains a retention time of -15% to 115% or a range that attains a retention time of -20% to 120% when the retention time is converted to a methylation rate. When the plot of the first derivative value has a shoulder peak, a second derivative value is further calculated and plotted to determine whether or not the target DNA is DNA derived from a tissue having renal cell carcinoma having poor prognosis.

The method of the present invention using the derivative value of the chromatography detection signal enables accurate prognosis determination even for renal cell carcinoma whose prognosis is difficult to determine by conventional examination. Thus, the method of the present invention enables accurate determination of whether or not cancer in a renal cell carcinoma patient or a patient suspected of having renal cell carcinoma is highly malignant renal cell carcinoma with poor prognosis. According to the present invention, a more appropriate plan of treatment can be made for renal cell carcinoma patients, and by extension, the survival rate of patients can be improved.

### [Examples]

Hereinafter, the present invention will be described in detail with reference to Examples. However, the present invention is not intended to be limited by Examples given below.

### [Patient and tissue sample]

109 cancer tissue (T) samples and corresponding 107 noncancerous renal cortical tissue (N) samples were obtained from samples operatively excised from 110 patients affected by primary clear cell renal cell carcinoma. No remarkable histological change was observed in the N samples. These patients did not receive preoperative treatment. These patients had undergone nephrectomy at the National Cancer Center. The patients consisted of 79 males and 31 females with an average age of 62.8 ± 10.3 years old (mean ± standard deviation, 36 to 85 years old).

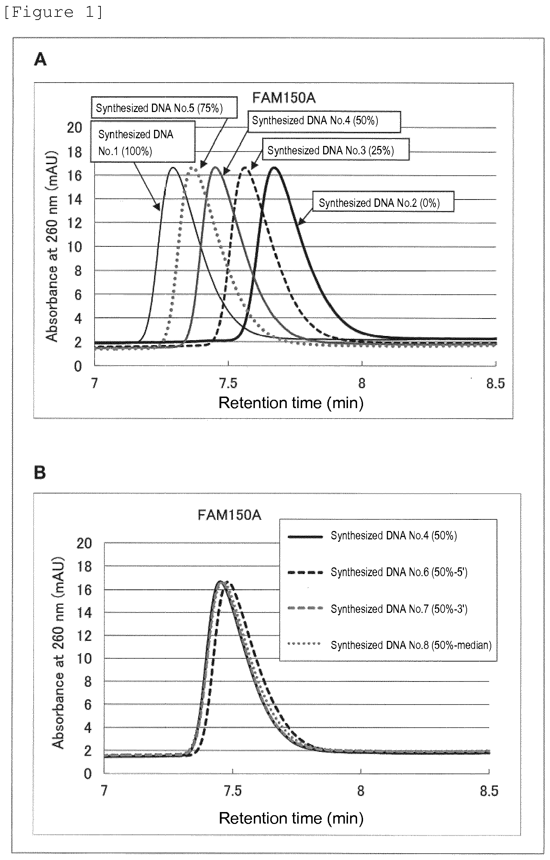

The histological diagnosis of the samples was conducted according to the classification of WHO (see Eble, J.N. et al., "Renal cell carcinoma, WHO Classification of Tumours. Pathology and Genetics of Tumours of the Urinary System and Male Genital Organs", 2004, IARC Press, Lyon, p. 10-43, Figure 1).

The histological grades of all tumors were evaluated according to the criterion described in "Fuhrman, S.A. et al., Am. J. Surg. Pathol., 1982, Vol. 6, p. 655-663". TNM classification followed "Sobin, L.H. et al., UICC, TNM Classification of Malignant Tumours, 6th edition, 2002, Wiley-Liss, New York, p. 193-195".

The criteria established for hepatocellular cancer (HCC) were adopted as criteria for the macroscopic classification of renal cell carcinoma (see Non Patent Literatures 4 to 6). Type 3 (contiguous multinodular type) HCC has a lower histological degree of differentiation and a higher occurrence rate of intrahepatic metastasis than those of HCC of type 1 (single nodular type) and type 2 (single nodular type with extranodular growth) (see Kanai, T. et al., Cancer, 1987, Vol. 60, p. 810-819).

The presence or absence of blood vessel invasion was examined by microscopically observing slides provided with hematoxylin-eosin staining and Elastica van Gieson staining. The presence or absence of tumor thrombus in the main trunk of the renal vein was examined by macroscopic observation.

This study was conducted after obtainment of written informed consent from all of the patients to be studied here. Also, this study was carried out under the approval of the Ethical Committee of the National Cancer Center.

### [Reference Example 1] Determination of CIMP negativity or positivity by conventional method

The determination of CIMP negativity or positivity by a conventional method was conducted according to the MassARRAY method described in Patent Literature 4 (Example 5). The DNA methylation levels at the CpG sites of 17 genes (FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2) (Tables 1 to 4) were detected by the MassARRAY method, which is one of the methods for detecting methylated DNA.

The MassARRAY method is a method which involves amplifying DNA after bisulfite treatment, transcribing the amplification product to RNA, further cleaving the RNA with RNase in a base-specific manner, and then detecting the difference in molecular weight between a methylated DNA fragment and an unmethylated DNA fragment using a mass spectrometer.

First, MassARRAY primers were designed for CpG islands containing the CpG sites using EpiDesigner (primer design software for MassARRAY manufactured by Sequenom).

In order to eliminate the influence of bias in PCR, 3 DNA polymerases and conditions involving approximately 4 annealing temperatures on average per primer set were combined for PCR runs to determine the optimum PCR conditions with good quantitative performance. Furthermore, all of the analyte CpG sites contained in PCR target sequences were confirmed to exhibit good quantitative performance under the adopted PCR conditions. The MassARRAY analysis was carried out on 102 renal cell carcinoma specimens among the 109 renal cell carcinoma tissue samples.

First, fresh frozen tissue samples obtained from the patients were each treated with phenol-chloroform and subsequently dialyzed to extract high-molecular-weight DNA (see Sambrook, J. et al., Molecular Cloning: A Laboratory Manual, 3rd edition, Cold Spring Harbor Laboratory Press, NY, p. 6.14-6.15). Then, 500 ng of the DNA was treated with bisulfite using EZ DNA Methylation-Gold(TM) kit (manufactured by Zymo Research Corp.). The bisulfite-treated genomic DNA was amplified by PCR, followed by *in vitro* transcription reaction. Subsequently, the obtained RNA was specifically cleaved at uracil sites with RNase to form fragments differing in length according to the presence or absence of methylation in the genomic DNA of each sample. Then, the obtained RNA fragments were subjected to mass spectrometry by MALDI-TOF MAS (MassARRAY Analyzer 4, manufactured by Sequenom) capable of detecting the difference in the mass of one base. The obtained mass spectrometry result was aligned with the reference sequence using analysis software (EpiTYPER, manufactured by Sequenom). The methylation level was calculated from the mass ratio between the RNA fragment derived from methylated DNA and the RNA fragment derived from unmethylated DNA. The sequences of the primers used in this analysis and the sequences of the PCR amplification products obtained using sets of these primers are shown in Tables 5 and 6 and the Sequence Listing.

**[Table 5]**

| Primer set name | PCR product size | Forward primer | Reverse primer | Target sequence (sequence of PCR product) |
|---|---|---|---|---|
| SLC13A5_MA_10 | 500 | | | SEQ ID NO: 1 |
| SLC13A5_MA_13 | 463 | | | SEQ ID NO: 2 |
| SLC13A5_MA_15 | 384 | | | SEQ ID NO: 3 |
| FAM150A_MA_14 | 455 | | | SEQ ID NO: 4 |
| GRM6_MA_8 | 188 | | | SEQ ID NO: 5 |
| ZFP42_MA_2 | 196 | | | SEQ ID NO: 6 |
| ZNF154_MA_5 | 279 | | | SEQ ID NO: 7 |
| RIMS4_MA_9 | 402 | | | SEQ ID NO: 8 |

**[Table 6]**

| Target gene name | PCR product size | Forward primer | Reverse primer | Target sequence (sequence of PCR product) |
|---|---|---|---|---|
| TRH_MA_8 | 414 | | | SEQ ID NO:9 |
| ZNF540_MA_17 | 463 | | | SEQ ID NO:10 |
| PCDHAC1_MA_5 | 362 | | | SEQ ID NO:11 |
| PRAC_MA_2 | 264 | | | SEQ ID NO:12 |
| ZNF671_MA_8 | 428 | | | SEQ ID NO:1 3 |
| WNT3A_MA_9 | 348 | | | SEQ ID NO:1 4 |
| KHDRBS2_MA_19(rev) | 422 | | | SEQ ID NO:15 |
| ASCL2_MA_8 | 339 | | | SEQ ID NO:1 6 |

The DNA methylation levels of the CpG sites were detected for all of the MassARRAY analyte regions to differentiate between renal cell carcinoma having poor prognosis (CIMP-positive group: 14 specimens) and renal cell carcinoma having good prognosis (CIMP-negative group: 88 specimens).

### [Reference Example 2] Detection of methylated DNA by ion exchange chromatography

### (1) Preparation of anion exchange column

In a reactor equipped with a stirrer, a mixture of 200 g of tetraethylene glycol dimethacrylate (manufactured by Shin-Nakamura Chemical Co., Ltd.), 100 g of triethylene glycol dimethacrylate (manufactured by Shin-Nakamura Chemical Co., Ltd.), 100 g of glycidyl methacrylate (manufactured by Wako Pure Chemical Industries, Ltd.), and 1.0 g of benzoyl peroxide (manufactured by Kishida Chemical Co., Ltd.) was added to 2,000 mL of an aqueous solution containing 3 wt% of polyvinyl alcohol (manufactured by The Nippon Synthetic Industry Co., Ltd.). The reaction mixture was heated with stirring and polymerized at 80°C for 1 hour in the nitrogen atmosphere. Next, 100 g of ethyl methacrylate trimethylammonium chloride (manufactured by Wako Pure Chemical Industries, Ltd.) was dissolved as the hydrophilic monomer having the strong cationic group in ion exchange water. This solution was added to the reactor mentioned above. Similarly, the reaction mixture was polymerized with stirring at 80°C for 2 hours in the nitrogen atmosphere. The obtained polymer composition was washed with water and acetone to obtain coated polymer particles having, on the surface, a hydrophilic polymer layer having a quaternary ammonium group. The obtained coated polymer particles were found to have an average particle size of 10 µm by measurement using a particle size distribution measurement apparatus (AccuSizer 780, manufactured by Particle Sizing Systems).

10 g of the obtained coated polymer particles was dispersed in 100 mL of ion exchange water to prepare pre-reaction slurry. Subsequently, 10 mL of N,N-dimethylaminopropylamine (manufactured by Wako Pure Chemical Industries, Ltd.) was added to this slurry with stirring, and the mixture was reacted at 70°C for 4 hours. After the completion of the reaction, the supernatant was removed using a centrifuge (manufactured by Hitachi, Ltd., "Himac CR20G"), and the residue was washed with ion exchange water. After the washing, the supernatant was removed using a centrifuge. This washing with ion exchange water was further repeated four times to obtain a packing material for ion exchange chromatography having a quaternary ammonium group and a tertiary amino group on the surface of the substrate particles.

A stainless column (column size: inside diameter 4.6 mm × length 20 mm) of a liquid chromatography system was packed with the packing material for ion exchange chromatography.

### (2) Extraction and bisulfite treatment of genomic DNA

Fresh frozen tissue samples obtained from the patients were each treated with phenol-chloroform and subsequently dialyzed to extract high-molecular-weight DNA (see Sambrook, J. et al., Molecular Cloning: A Laboratory Manual, 3rd edition, Cold Spring Harbor Laboratory Press, NY, p. 6.14-6.15). 500 ng of the DNA was treated with bisulfite using EZ DNA Methylation-Gold(TM) kit (manufactured by Zymo Research Corp.).

### (3) PCR

The bisulfite-treated genomic DNA obtained in the preceding step (2) was amplified by PCR. The PCR was performed using a 25 µL of a reaction solution containing 10 ng of template DNA, GeneAmp 1 × PCR buffer (manufactured by Life Technologies Corp.), 200 µmol/L GeneAmp dNTP Mix (manufactured by Life Technologies Corp.), 0.75 U AmpliTaq Gold DNA Polymerase (manufactured by Life Technologies Corp.), and 0.25 µmol/L forward and reverse primers. The PCR involved initial thermal denaturation at 95°C for 5 minutes, followed by 35 cycles each involving 94°C for 30 seconds → 59°C (in the case of using F3-R3 primer) for 30 seconds → 72°C for 40 seconds, and subsequent elongation reaction at 72°C for 10 minutes. After the completion of the PCR, 5 µL of the reaction solution was mixed with 1 µL of a loading dye solution, then applied to a 3% agarose gel supplemented with ethidium bromide in advance, and electrophoresed. The PCR amplification product was observed to confirm that the PCR amplification product of interest was obtained. The sequence of each primer is shown in Table 7.

**[Table 7]**

| Target gene name | Product size | Target Seq |
|---|---|---|
| FAM150A_ MA_14 0% methylation | 384 | |
| FAM150A_ MA_14 100% methylation | 384 | |
| Primer | forward | GGGAGGATTTAGTAGGGTAATTGT (SEQ ID NO:51) |
| | reverse | ATACCCAACCCCAAACCTCTTC (SEQ ID NO:52) |

| | | |
|---|---|---|
| Primer binding sites are underlined. | | |

### (4) HPLC analysis

The anion exchange column prepared in Reference Example 2 was used in ion exchange chromatography under the following conditions to separate and detect each PCR amplification product obtained in the preceding step (3).
System: LC-20A series (manufactured by Shimadzu Corp.)
Eluent: eluent A: 25 mmol/L tris-HCl buffer solution (pH 7.5)
eluent B: 25 mmol/L tris-HCl buffer solution (pH 7.5) + 1 mol/L ammonium sulfate
Analysis time: 15 min
Elution method: the mixing ratio of eluent B was linearly increased under the following gradient conditions:
   0 min (40% eluent B) → 10 min (100% eluent B)
   Specimen: the PCR amplification product obtained in the step (3)
   Flow rate: 1.0 mL/min
   Detection wavelength: 260 nm
   Sample injection volume: 5 µL
   Column temperature: 70°C

### [Reference Example 3] Variation in chromatography retention time caused by DNA methylation rate

On the basis of the DNA sequence of a 384-bp region having 39 CpG sites in a FAM150A gene promoter, 8 DNAs differing in methylation rate were synthesized from DNA in which all of the 39 CpG sites were methylated (100% methylated DNA) through DNA in which none of the 39 CpG sites were methylated (0% methylated DNA). The 50% methylated DNA was synthesized as 3 patterns of DNA having a methylation position closer to the 5' end, closer to the 3' end, and closer to the center, respectively. The methylation rate of each synthesized DNA and the number of its methylated sites in the CpG island are shown in Table 8. The nucleotide sequence of the synthesized DNA was designed as a sequence after bisulfite treatment. Specifically, cytosine at sites other than the CpG sites and unmethylated forms of cytosine at the CpG sites were all replaced with thymine for synthesis.

**[Table 8]**

| Synthesized DNA No. | Methylation rate (position on CpG island) | The number of methylated CpG sites | The number of unmethylat ed CpG sites |
|---|---|---|---|
| 1 | 100% | 39 | 0 |
| 2 | 0% | 0 | 39 |
| 3 | 25% (random) | 10 | 29 |
| 4 | 50% (random) | 20 | 19 |
| 5 | 75% (random) | 30 | 9 |
| 6 | 50% (closer to 5' end) | 20 | 19 |
| 7 | 50% (closer to 3' end) | 20 | 19 |
| 8 | 50% (center) | 20 | 19 |

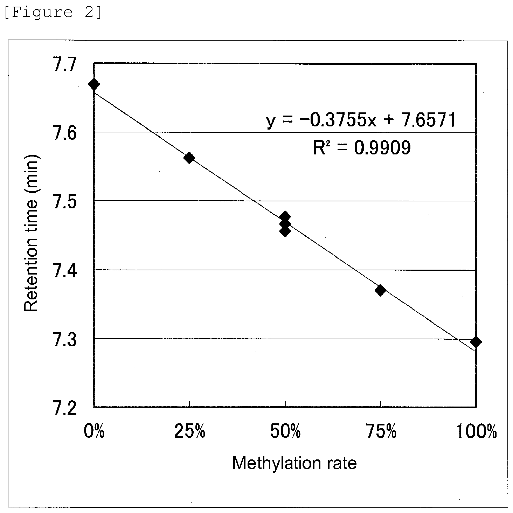

The 8 synthesized DNAs were subjected to PCR and HPLC analysis according to the procedures of Reference Examples 2(3) and 2(4). The HPLC chromatograms of synthesized DNA No. 1 (100% methylation), No. 2 (0% methylation), No. 3 (25% methylation), No. 4 (50% methylation), and No. 5 (75% methylation) are shown in Figure 1A. As shown in Figure 1A, reduction in retention time was found with increase in DNA methylation rate. Data obtained by plotting the methylation rates of the 8 DNAs vs. HPLC retention times is shown in Figure 2. As shown in Figure 2, the HPLC retention times exhibited very high correlation with the DNA methylation rates. The 50% methylated DNAs were confirmed to exhibit almost the same retention time, irrespective of their methylation positions (Figure 1B). This demonstrated that retention times are determined depending on methylation rates, irrespective of methylation positions in DNA. Thus, the methylation rate of CpG sites contained in sample DNA can be measured by measuring the HPLC retention time.

### [Example 1] Prognosis determination for renal cell carcinoma based on chromatography retention time

Genomic DNA was prepared from each of one CIMP-positive renal cell carcinoma specimen and one CIMP-negative renal cell carcinoma specimen determined as having CIMP-positive or -negative renal cell carcinoma in Reference Example 1. The DNA was subjected to bisulfite treatment, PCR, and HPLC according to the procedures of Reference Examples 2(2) to 2(4). In PCR, a 384-bp region in a FAM150A gene promoter was amplified.

Furthermore, each DNA having a methylation rate of 0% (negative control) or 100% (positive control) in the PCR amplification region was also analyzed by HPLC according to the same procedures as above.

The HPLC chromatograms obtained from the CIMP-positive and CIMP-negative specimens are shown in Figure 3. Figure 3 also shows the chromatograms of the unmethylated DNA (negative control) and the 100% methylated DNA (positive control). Figure 3A is the chromatograms of the CIMP-positive specimen (CIMP-positive specimen 01), and a peak differing in retention time from the peak of the unmethylated DNA (negative control) appeared clearly, indicating the presence of methylated DNA. Figure 3B is the chromatogram of the CIMP-negative specimen (CIMP-negative specimen 01), and its peak was hardly able to be discriminated from the peak of the unmethylated DNA (negative control) at their retention times, indicating that highly methylated DNA was almost absent.

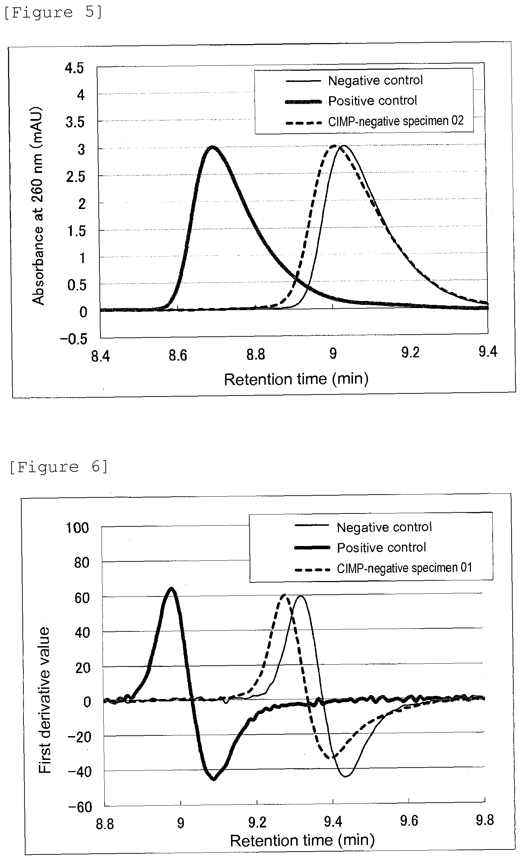

Figure 4 shows the chromatogram of another CIMP-positive specimen (CIMP-positive specimen 02), and Figure 5 shows the chromatogram of another CIMP-negative specimen (CIMP-negative specimen 02). In Figure 4, as with Figure 3A, bimodal peaks appeared clearly, indicating the presence of methylated DNA. In Figure 5, a unimodal peak analogous to the peak of the negative control was obtained, indicating that highly methylated DNA was almost absent, as with Figure 3B.

### [Example 2] Prognosis determination for renal cell carcinoma based on derivative value of chromatography detection signal

Genomic DNA was prepared from each of one CIMP-negative renal cell carcinoma specimen actually having good prognosis, one CIMP-positive renal cell carcinoma specimen actually having poor prognosis, and one CIMP-negative renal cell carcinoma (false negative) specimen actually having poor prognosis, determined as having CIMP-positive or -negative renal cell carcinoma in Reference Example 1. The DNA was subjected to bisulfite treatment, PCR, and HPLC according to the procedures of Reference Examples 2(2) to 2(4). In PCR, a 384-bp region in a FAM150A gene promoter was amplified.

A first derivative value (signal curve slope) of the obtained HPLC detection signal with respect to the retention time was calculated using Microsoft(R) Excel(R). Unless otherwise specified, the range on the X-axis (i.e., the range of the retention time) for determining a maximum or a minimum in the plot of the derivative value was set to the range from the peak top retention time of the HPLC detection signal from unmethylated (0% methylated) DNA (negative control) to the peak top retention time of the HPLC detection signal from 100% methylated DNA (positive control). In other words, the range was from the retention time at which Y = 0 between the maximum and the minimum in the first derivation plot of the unmethylated DNA (negative control) to the retention time at which Y = 0 between the maximum and the minimum in the first derivation plot of the 100% methylated DNA (positive control).

Figure 6 shows the plot of the first derivative value of the HPLC detection signal obtained from the CIMP-negative specimen having good prognosis (CIMP-negative specimen 01) with respect to the retention time. Figure 6 also shows the first derivative values of the detection signals obtained from the negative control and the positive control. The data from the CIMP-negative specimen (CIMP-negative specimen 01) was represented by a curve having one maximum at between approximately 9.2 and 9.3 minutes and decreasing from the maximum to 0 at between approximately 9.3 and 9.4 minutes. This is similar to the data on the negative control in the shape of the plot and the retention time, indicating that the original HPLC detection signal was a signal having a unimodal peak analogous to the peak of the negative control.

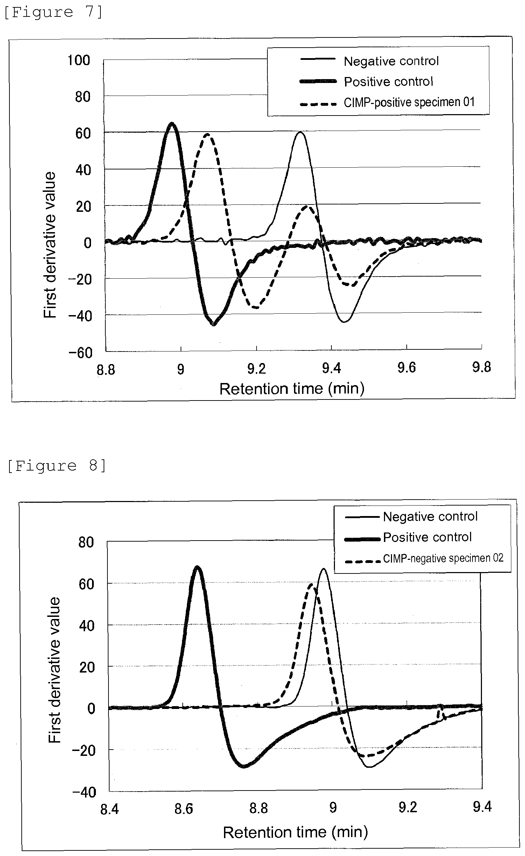

Figure 7 shows the plot of the first derivative value of the HPLC detection signal obtained from the CIMP-positive specimen having poor prognosis (CIMP-positive specimen 01) with respect to the retention time. This was represented by a curve having two or more maximums and, in this point, differed evidently from the first derivative values of the CIMP-negative specimens in Figure 6 and Figure 8 (mentioned later). The first maximum resided at between approximately 9.0 and 9.1 minutes, and the second maximum resided at between approximately 9.3 and 9.4 minutes. The minimum between the first maximum and the second maximum was positioned at approximately 9.2 minutes. This indicates that the original HPLC detection signal was a signal having bimodal peaks and having a peak at a time shorter than that of the peaks of the CIMP-negative specimens.

Figure 8 shows the plot of the first derivative value of the HPLC detection signal obtained from another CIMP-negative specimen having good prognosis (CIMP-negative specimen 02) with respect to the retention time. Figure 8 also shows the derivative values of the detection signals obtained from the negative control and the positive control. The data from the CIMP-negative specimen (CIMP-negative specimen 02) was represented by a curve having one maximum at between approximately 8.9 and 9.0 minutes and decreasing from the maximum to 0 at between approximately 9.0 and 9.1 minutes. This is similar to the data on the negative control in the shape of the plot and the retention time, indicating that the original HPLC detection signal was a signal having a unimodal peak analogous to the peak of the negative control.

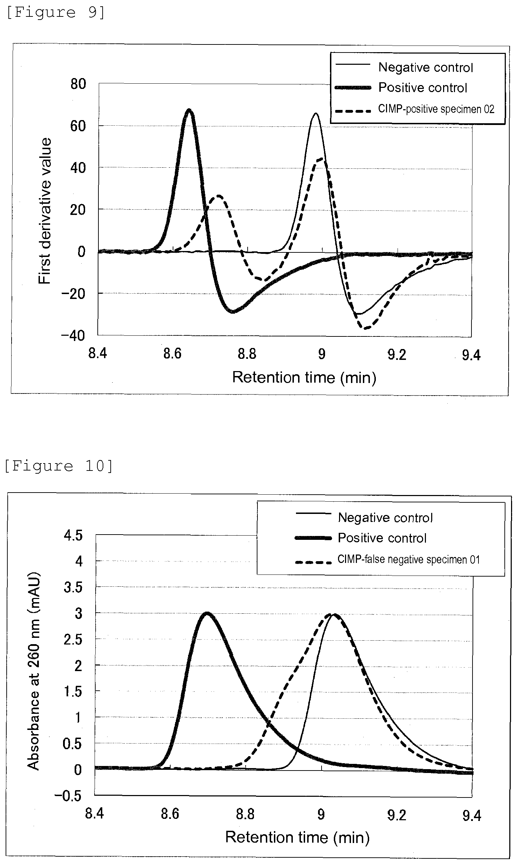

Figure 9 shows the plot of the first derivative value of the HPLC detection signal obtained from another CIMP-positive specimen having poor prognosis (CIMP-positive specimen 02) with respect to the retention time. This was represented by a curve having two or more maximums and, in this point, differed evidently from the first derivative values of the CIMP-negative specimens in Figures 6 and 8. The first maximum resided at between approximately 8.7 and 8.8 minutes, and the second maximum resided at approximately 9.0 minutes. The minimum between the first maximum and the second maximum was positioned at between approximately 8.8 and 8.9 minutes. This indicates that the original HPLC detection signal was a signal having bimodal peaks and having a peak at a time shorter than that of the peaks of the CIMP-negative specimens.

Thus, it was revealed that a CIMP-negative specimen and a CIMP-positive specimen can be distinguished very easily therebetween by calculating the derivative value of the chromatography detection signal.

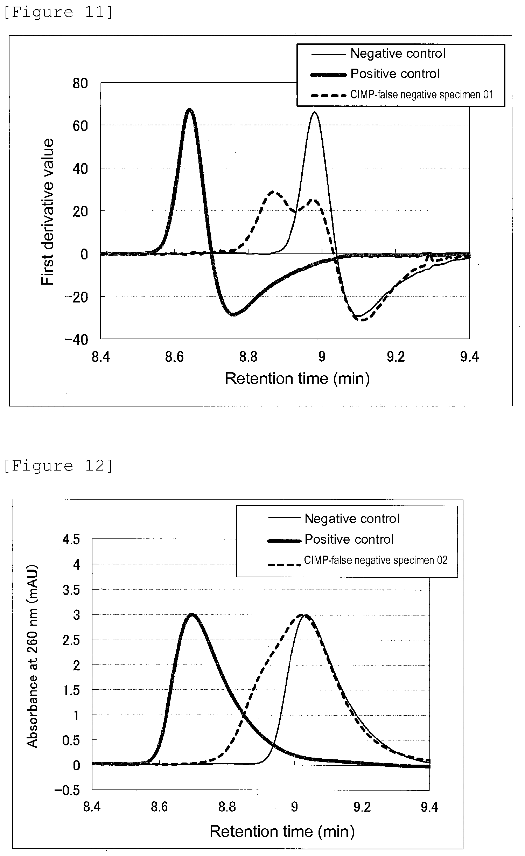

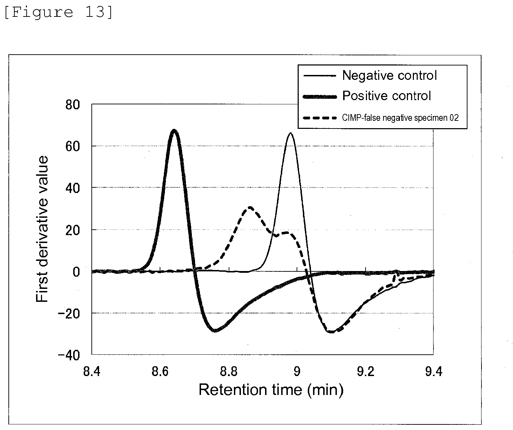

Figure 10 shows the chromatogram of the HPLC detection signal obtained from the renal cell carcinoma specimen actually having poor prognosis (CIMP-false negative specimen 01), though determined as having CIMP-negative renal cell carcinoma in the determination of CIMP negativity or positivity by the conventional method shown in Reference Example 1. Figure 11 shows the plot of the first derivative value of the HPLC detection signal of Figure 10 with respect to the retention time. Figure 12 shows the chromatogram of the HPLC detection signal obtained from another CIMP-false negative specimen (CIMP-false negative specimen 02). Figure 13 shows the plot of the first derivative value of the HPLC detection signal of Figure 12 with respect to the retention time. The plots of Figures 11 and 13 were each represented by a curve having two or more maximums, and, in this point, differed evidently from the first derivative values of the CIMP-negative specimens in Figures 6 and 8 and rather, were similar to the first derivative values of the CIMP-positive specimens in Figures 7 and 9. Thus, a specimen determined as having CIMP-false negative renal cell carcinoma by the conventional method was able to be correctly determined as a specimen having poor prognosis by analyzing the derivative value of the HPLC detection signal. Thus, a patient with poor prognosis was able to be found. According to the method of the present invention, prognosis determination for renal cell carcinoma can be achieved highly accurately.

## Claims

1. A method for determining a tissue having renal cell carcinoma having poor prognosis, comprising:
(1) subjecting sample DNA to ion exchange chromatography, wherein the sample DNA is obtained by treating target genomic DNA prepared from a renal tissue of a subject with bisulfite, followed by PCR amplification, wherein the subject is a renal cell carcinoma patient or a patient whose renal cell carcinoma has been treated by surgical operation, and who is in need of determination of the prognosis of the renal cell carcinoma;
(2) calculating a derivative value of a detection signal of the chromatography; and
(3) determining the renal tissue as being a tissue having renal cell carcinoma having poor prognosis when the derivative value calculated in the step (2) has two or more maximums,
wherein the target genomic DNA comprises a CpG island in at least one gene selected from the group consisting of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2.

2. A method for determining the prognosis of a renal cell carcinoma patient, comprising:
(1) subjecting sample DNA to ion exchange chromatography, wherein the sample DNA is obtained by treating target genomic DNA prepared from a renal tissue of a subject with bisulfite, followed by PCR amplification, wherein the subject is a renal cell carcinoma patient or a patient whose renal cell carcinoma has been treated by surgical operation, and who is in need of determination of the prognosis of the renal cell carcinoma;
(2) calculating a derivative value of a detection signal of the chromatography; and
(3) determining the subject as being a patient with renal cell carcinoma having poor prognosis when the derivative value calculated in the step (2) has two or more maximums,
wherein the target genomic DNA comprises a CpG island in at least one gene selected from the group consisting of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2.

3. A method for obtaining data for determining a tissue having renal cell carcinoma, comprising:
(1) subjecting sample DNA to ion exchange chromatography, wherein the sample DNA is obtained by treating target genomic DNA prepared from a renal tissue of a subject with bisulfite, followed by PCR amplification, wherein the subject is a renal cell carcinoma patient or a patient whose renal cell carcinoma has been treated by surgical operation, and who is in need of determination of the prognosis of the renal cell carcinoma;
(2) calculating a derivative value of a detection signal of the chromatography; and
(3) obtaining whether or not the derivative value calculated in the step (2) has two or more maximums as data for determining whether or not the tissue is a tissue having renal cell carcinoma having poor prognosis,
wherein the target genomic DNA comprises a CpG island in at least one gene selected from the group consisting of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2.

## Patentansprüche

1. Verfahren zur Bestimmung, dass ein Gewebe ein Nierenzellkarzinom mit schlechter Prognose aufweist, umfassend:
(1) Unterziehen einer Proben-DNA einer lonenaustauschchromatographie, wobei die Proben-DNA durch Behandeln einer genomischen Ziel-DNA, die aus einem Nierengewebe eines Patienten hergestellt wurde, mit Bisulfit, gefolgt von einer PCR-Amplifikation, erhalten wird; wobei der Proband ein Patient mit einem Nierenzellkarzinom ist oder ein Patient, dessen Nierenzellkarzinom mittels eines chirurgischen Eingriffs behandelt wurde und der die Bestimmung der Prognose des Nierenzellkarzinoms benötigt,
(2) Berechnen eines Ableitungswerts eines Detektionssignals der Chromatographie und
(3) Bestimmen des Nierengewebes als Gewebe, das ein Nierenzellkarzinom mit schlechter Prognose aufweist, wenn der in Schritt (2) berechnete Ableitungswert zwei oder mehr Maxima aufweist, wobei die genomische Ziel-DNA eine CpG-Insel in wenigstens einem Gen umfasst, ausgewählt aus der Gruppe, bestehend aus FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5 und NKX6-2.

2. Verfahren zur Bestimmung der Prognose eines Patienten mit Nierenzellkarzinom, umfassend:
(1) Unterziehen einer Proben-DNA einer lonenaustauschchromatographie, wobei die Proben-DNA durch Behandeln einer genomischen Ziel-DNA, die aus einem Nierengewebe eines Patienten hergestellt wurde, mit Bisulfit, gefolgt von einer PCR-Amplifikation, erhalten wird; wobei der Proband ein Patient mit einem Nierenzellkarzinom ist oder ein Patient, dessen Nierenzellkarzinom mittels eines chirurgischen Eingriffs behandelt wurde und der die Bestimmung der Prognose des Nierenzellkarzinoms benötigt,
(2) Berechnen eines Ableitungswerts eines Detektionssignals der Chromatographie und
(3) Bestimmen, dass der Proband ein Patient ist, der ein Nierenzellkarzinom mit schlechter Prognose aufweist, wenn der in Schritt (2) berechnete Ableitungswert zwei oder mehr Maxima aufweist, wobei die genomische Ziel-DNA eine CpG-Insel in wenigstens einem Gen umfasst, ausgewählt aus der Gruppe, bestehend aus FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5 und NKX6-2.

3. Verfahren zur Gewinnung von Daten zur Bestimmung eines Gewebes mit Nierenzellkarzinom, umfassend:
(1) Unterziehen einer Proben-DNA einer lonenaustauschchromatographie, wobei die Proben-DNA durch Behandeln einer genomischen Ziel-DNA, die aus einem Nierengewebe eines Patienten hergestellt wurde, mit Bisulfit, gefolgt von einer PCR-Amplifikation, erhalten wird; wobei der Proband ein Patient mit einem Nierenzellkarzinom ist oder ein Patient, dessen Nierenzellkarzinom mittels eines chirurgischen Eingriffs behandelt wurde und der die Bestimmung der Prognose des Nierenzellkarzinoms benötigt,
(2) Berechnen eines Ableitungswerts eines Detektionssignals der Chromatographie und
(3) Erhalt, als Daten, ob der in Schritt (2) berechnete Ableitungswert zwei oder mehr Maxima aufweist oder nicht, zur Bestimmung, ob das Gewebe ein Gewebe ist, das ein Nierenzellkarzinom mit schlechter Prognose aufweist oder nicht, wobei die genomische Ziel-DNA eine CpG-Insel in wenigstens einem Gen umfasst, ausgewählt aus der Gruppe, bestehend aus FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5 und NKX6-2.

## Revendications

1. Procédé pour déterminer qu'un tissu ayant un carcinome à cellules rénales a un mauvais pronostic, comprenant :
(1) la soumission d'un échantillon d'ADN à une chromatographie par échange d'ions, dans lequel l'échantillon d'ADN est obtenu par traitement d'ADN génomique cible préparé à partir d'un tissu rénal d'un sujet avec un bisulfite, suivi d'une amplification PCR, dans lequel le sujet est un patient souffrant d'un carcinome à cellules rénales ou un patient dont le carcinome à cellules rénales a été traité par une opération chirurgicale, et pour lequel il est nécessaire de déterminer le pronostic du carcinome à cellules rénales ;
(2) le calcul d'une valeur dérivée d'un signal de détection de la chromatographie ; et
(3) la détermination que le tissu rénal est un tissu ayant un carcinome à cellules rénales ayant un mauvais pronostic quand la valeur dérivée calculée dans l'étape (2) a deux ou plus deux maxima,
dans lequel l'ADN génomique cible comprend un îlot CpG dans au moins un gène choisi dans le groupe constitué par FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, et NKX6-2.

2. Procédé pour déterminer le pronostic d'un patient souffrant d'un carcinome à cellules rénales, comprenant :
(1) la soumission d'un échantillon d'ADN à une chromatographie par échange d'ions, dans lequel l'échantillon d'ADN est obtenu par traitement d'ADN génomique cible préparé à partir d'un tissu rénal d'un sujet avec un bisulfite, suivi d'une amplification PCR, dans lequel le sujet est un patient souffrant d'un carcinome à cellules rénales ou un patient dont le carcinome à cellules rénales a été traité par une opération chirurgicale, et pour lequel il est nécessaire de déterminer le pronostic du carcinome à cellules rénales ;
(2) le calcul d'une valeur dérivée d'un signal de détection de la chromatographie ; et
(3) la détermination que le sujet est un patient avec un carcinome à cellules rénales ayant un mauvais pronostic quand la valeur dérivée calculée dans l'étape (2) a deux ou plus deux maxima,
dans lequel l'ADN génomique cible comprend un îlot CpG dans au moins un gène choisi dans le groupe constitué par FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, et NKX6-2.

3. Procédé pour obtenir des données pour la détermination d'un tissu ayant un carcinome à cellules rénales, comprenant :
(1) la soumission d'un échantillon d'ADN à une chromatographie par échange d'ions, dans lequel l'échantillon d'ADN est obtenu par traitement d'ADN génomique cible préparé à partir d'un tissu rénal d'un sujet avec un bisulfite, suivi d'une amplification PCR, dans lequel le sujet est un patient souffrant d'un carcinome à cellules rénales ou un patient dont le carcinome à cellules rénales a été traité par une opération chirurgicale, et pour lequel il est nécessaire de déterminer le pronostic du carcinome à cellules rénales ;
(2) le calcul d'une valeur dérivée d'un signal de détection de la chromatographie ; et
(3) l'obtention que la valeur dérivée calculée dans l'étape (2) a ou non deux maxima en tant que données pour déterminer si oui ou non le tissu est un tissu ayant un carcinome à cellules rénales ayant un mauvais pronostic,
dans lequel l'ADN génomique cible comprend un îlot CpG dans au moins un gène choisi dans le groupe constitué par FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, et NKX6-2.


### SEQUENCE LISTING

<110> NATIONAL CANCER CENTER SEKISUI MEDICAL CO., LTD.
<120> Method of determinating prognosis of renal cell carcinoma
<130> DC0101
<150> JP 2015-173311
   <151> 2015-09-02
<160> 52
<170> PatentIn version 3.1
<210> 1
   <211> 500
   <212> DNA
   <213> Homo sapiens
<220>
   <223> SLC13A5_MA_10
<400> 1
   gaaggacttg aacttggaga catagctcag cgccgaggcc atcgcgcggg agggagactg      60
   gcgggcgaga cgagtgaggg gcagctagag gcgccgcggg cttaagaagg ggccacagtc     120
   cccggggatt ggggaggggg cggtgacaac tccgccccgc acgggggcgc ctccccgcgg     180
   ccctggggcg gggccacccc tcggggtctg tgggacgcgc ctgcccccaa ttctgccacc     240
   cggcggcggt gggaggcgtc tttggactcc aacgcttcgg gccagccctc taggggcagc     300
   ctgggcccta gcatctcgcg ctgtccaagc ctctcctgcg ctgccgaggc agaggtgcgt     360
   cccggggctg ccaagcgggg cgtgttttgg tcactggtgc tgcccgcttt ggcgtaaggc     420
   gccctcccgc gtccgcatct gctctttcct gggctctgaa gggtcccgga tgaaactctc     480
   tgcaggcctc tgggtctctc                                                 500
<210> 2
   <211> 463
   <212> DNA
   <213> Homo sapiens
<220>
   <223> SLC13A5_MA_13
<400> 2
   ctttcctggg ctctgaaggg tcccggatga aactctctgc aggcctctgg gtctctcagg     60
   tctatctccc cgatctccct ctcctttcca tctccttact tccgcccctc cggtgtctct    120
   ccgagaggtg tccccccacg ccccgcgccc tccgcaccgc gggcctcgct tcccggtccc    180
   ccctggcttc ctcgccacgt ccgccccact ctaggtgcag gacccctttt ccccgctcgc    240
   actctccggc ccggagctcc tgggcgatcg cacagggaag cgaggccact gtcctcctct    300
   gtcccagggg ctgtcgcgct ccagtggacg ctgcaccccg cagacgcccg gcgggcagat    360
   gcggacacgc gtcttggagg ggccccaccg agcctcagca gccgcagctg cccgcccgac    420
   ccaggtcaga gggaaacgga gctctagaga ggaagggaca caa                      463
<210> 3
   <211> 384
   <212> DNA
   <213> Homo sapiens
<220>
   <223> SLC13A5_MA_15
<400> 3
   cctcctctgt cccaggggct gtcgcgctcc agtggacgct gcaccccgca gacgcccggc     60
   gggcagatgc ggacacgcgt cttggagggg ccccaccgag cctcagcagc cgcagctgcc    120
   cgcccgaccc aggtcagagg gaaacggagc tctagagagg aagggacaca actaaggcga    180
   cactgagaca gtcgcccatg tattcattca gcccgccagg caacagacag gtgccgagca    240
   cctcttctcc gcgaggccct gttttgggca ctggagacac acggatgcaa agacatcccc    300
   acctctgtga ttttcttctt tcctctcctc tgcctgcctc tcattctgca gcttcctttt    360
   ggggagtctc atccatgctg gtgg                                           384
<210> 4
   <211> 455
   <212> DNA
   <213> Homo sapiens
<220>
   <223> FAM150A_MA_14
<400> 4
   gggaggaccc agtagggtaa ctgccgcgtc gccccggcgg ttctccctgg gctctgtctc     60
   ccgccgcctc caccccccga gcctcggggt ccgtcacggc ttcccctggc tggcggggtc    120
   agtagaaccc gcggcgccta ggtccggacg gaaaaaagca gggccggggt gcggcctgga    180
   tgagcggaga tctccgcgcc ttgggctcaa aggtgcgggg tgcgctctgc tgccgagccc    240
   ctgctcgctc aggaacactg gccacgccgt cacgccagcc gcccctgccc caggtctgga    300
   ggcccgacct gctctcctag gcgcagcacc gcgttctctt ccgcgtgggg gagcggcggg    360
   cggaagaggt ctggggctgg gcaccgggga cacgcgccca gctcccctgg cctccctggg    420
   gggagtggcc ggtttcagtg cttccccagg tgaaa                              455
<210> 5
   <211> 188
   <212> DNA
   <213> Homo sapiens
<220>
   <223> GRM6_MA_8
<400> 5
   ggttcaggac aagtctgtga cagatgcggc cgaggccctg agcgagagag gatttaagga     60
   ttctagggag ggatgagaga ccgctccgag ggtggagacc cctcctgagt gtggggggtg    120
   gcggtgctgg ctctccccgc atctccttcc cctccctctc ccaatctctg ggtctgtttt    180
   cctgtttt                                                             188
<210> 6
   <211> 196
   <212> DNA
   <213> Homo sapiens
<220>
   <223> ZFP42_MA_2
<400> 6
   gagctgatgg gtggctgtag cctgattaga ccgcgtcagt ccggagggtg ggtcttggga     60
   gggggcgcag ggcagtccac gtttccactg cagtttctcc tttgttttac gtttgggagg    120
   aggtggcatt ggaaatagca gagtgcttcg cggtaacagg ggtgagtctt gtttcatgga    180
   acttttttca aatggg                                                    196
<210> 7
   <211> 279
   <212> DNA
   <213> Homo sapiens
<220>
   <223> ZNF154_MA_5
<400> 7
   ggtgaacaca cctcagagaa gctaaaatgg ccgccacgaa gaggcccccc caaaagtccc     60
   gtcctttctt tttgtgactc tcaaggaaag tcggttttct gagctcttac tggcttagta    120
   gcgtggcgtt caacgcagag cattctaggt aatgtagttt tcatagatcc cgaggtgggt    180
   gccggggacc ctttgcacca acctcttgga gtaaaagcga agctccaggg cgctgggcga    240
   tgagaaatgg cttatccaag tcctagggca gtggaggga                            279
<210> 8
   <211> 402
   <212> DNA
   <213> Homo sapiens
<220>
   <223> RIMS4_MA_9
<400> 8
   ggagccccag cccatgaggg aaggagagga gagataaatg ggggcgctca aggcctgggg     60
   cgccgggcag gggtcttggg cagggatcct ctggatgtgg ccaagacaaa gatggagagg    120
   taaggtctgc gcgccacctc caatggcggg gggcgcgtcg gagccccagg ggtgggacgg    180
   ccaaagccca gggcttgaag agtgggcaca ttcaggagac tcagggaggg tggcaggtcg    240
   gctccaggga cgaggcaagg ggcctccaat aggcgcgggt gaggagggag atgggtcctg    300
   gcgacccaaa gggcccacct gcgggaaagg tgaatgcaga caatctcggg gtccctgggg    360
   gagaaggcca gaaggtagcg catcctggag accctggggt cc                       402
<210> 9
   <211> 414
   <212> DNA
   <213> Homo sapiens
<220>
   <223> TRH_MA_8
<400> 9
   aacagatctc cagaggtggt gcagaaacga ccccgcgccg gcgccccatc ctgcggccag     60
   tgcctccgcg ccccggctcc ggtccccacc gtccccgccc cagatttccg gaggagcagg    120
   cgggcggggt cccgcggggc cggctgccgt cagcgcccct tcccggcggc cgcgacccct    180
   ccccgctgac ctcactcgag ccgccgcctg gcgcagatat aagcggcggc ccatctgaag    240
   agggctcggc aggcgcccgg ggtcctcagc gctgcagact cctgacctgc cgactgcgga    300
   tcccgagtcc ccggatcccg gacccatcct gtggagccca ctcctggcag gtaaccgccc    360
   caacccctct ccttccgcag acggtgtccg ggagcactgg aaagggagcc ctct          414
<210> 10
   <211> 463
   <212> DNA
   <213> Homo sapiens
<220>
   <223> ZNF540_MA_17
<400> 10
   gggcagggca gaaccaggct aaagaaacgt ccagcgtagc ttcaaggatg caccgcgtga     60
   tccctatcgg atctccccga cgcgtcaggc ctgcctagac ggtgctggga gcgcgtctcc    120
   ttgaacgttg tcccgcctgg gattgcgagg taggtaccgc ctgcctgtgt gtaccggggc    180
   tgctgtctcc ggggaggggc ttctggcgga caggagaacc aagcagcctc aggagctgcc    240
   tgggtgtgtg tgtttctgtg cgagtgttgc atatctctgt gtgtgtttct gtgcaagtgt    300
   tgcatgtctg tgtgtgtggg gggggtggtg tctggtgaaa agaatgtgtc tcgtgcggtg    360
   gagcgccgtt tctctgtagt ccgcgggctc tcttatgcgc cctcttgtgg tcccaagtgt    420
   gcttttcttg tttttcgctt tcttgggggt cattgatttc agc                      463
<210> 11
   <211> 362
   <212> DNA
   <213> Homo sapiens
<220>
   <223> PCDHAC1_MA_5
<400> 11
   tggcagctcc tgggatacaa gagggtgcag gacagacttc aacccgcagc aggatccagc     60
   gcggaaagct ctgcagcagg atccagcgcg gaaagccccc cgcagcactt ctttcggggg    120
   gctcctgttt ccttaagcct agaaggtgtg gtcgctcacg ttcaccgtcc cgcctctcgc    180
   cgcctccgct cggcagctcc acgctgagtc ccgccctctc cgccggagag gtgcgccggg    240
   gtcagagcgc cgggacccga cgcgcggctc ccaaagggcg gcaggaagag cccagctggg    300
   ctcagccaca gttatcagca atctgcgggc agaggatgtg gaggttaaga tctgggcagc    360
   ct                                                                   362
<210> 12
   <211> 264
   <212> DNA
   <213> Homo sapiens
<220>
   <223> PRAC_MA_2
<400> 12
   ggtgaaagcc tgctgctcac ccttcccttg tttcccaaaa cttctgaagg ctcccaaatt     60
   cctgggagac cctctcccag ggcctcctga tgcagctacc atactgagcg atccgtcgat    120
   aacgcccttg gcccaccgat cagtttacct tattagagag aaaagcactc ttggaggtag    180
   taagatgggc cggtccttga tctgagaaat gggcgcacaa catcgctgtt ctctctgcaa    240
   aggtggggac cagaatccag cttg                                           264
<210> 13
   <211> 428
   <212> DNA
   <213> Homo sapiens
<220>
   <223> ZNF671_MA_8
<400> 13
   tgggacacag gggctgcagg cactttacga ttcggagtcg gagaaagggt gactgagggc     60
   ccggaggacg cagcacccac ccgcgcggag tccgttagct ccgccatagg accgtgggcg    120
   cggacagctg ccgggagcgg caggcgtctc gatcggggac gcaggcactt ccgtccctgc    180
   agagcatcag acgcgtctcg ggacactggg gacaacatct cctccgcgct ttcccaacac    240
   ctccacctgc ggcccacaca agcgttacag aaccccggcc agggacagcc tgacagaaac    300
   aaaatgtccg ctacaaggag gagccggaag tcccgcccac gcaccccccg caggcactga    360
   aacacccctc tcctgggccc tcattgggta tgcaacgtat aggtttgtgg gtagagtgtg    420
   gttcccac                                                             428
<210> 14
   <211> 348
   <212> DNA
   <213> Homo sapiens
<220>
   <223> WNT3A_MA_9
<400> 14
   gtccacctgg caatgagggg ctgctgtaga gagagttaag ggtgagttaa gcacggggtg     60
   tgaggggctc caggaccctc aatcagaaag cgctgtgctg cgccctccac accagaaaag    120
   gcgcgttccg tgagaccctc cccagcctgg cgatggaagt gcagataaac caaaggaagg    180
   gtcccgaaag gtctctctca ggcctcccac ctccactgca catatcctgt gggaggggga    240
   acggtggcca cactttcgcc agggcttgtg atccctcaga gccctcacca agcaaggatc    300
   accccagttc cgaattaagg gcgctctgag atgcccaaga ttgaggaa                 348
<210> 15
   <211> 422
   <212> DNA
   <213> Homo sapiens
<220>
   <223> KHDRBS2_MA_19(rev)
<400> 15
   cctggcacca ccaccaatga gtggttggcg gaggtgggcc gcgtcctgtt cccgcctctc     60
   cagttaaggc cgctggtgtg agccggggct ctgcgcgagc gagggacgac ggaagggacg    120
   ggcaggtgtg ggcgcggggc cacgcagccc gacggcggga gtcgcaggtg ctgggtgcat    180
   gggccagtga ggacgcacag agatccctcg ccgcgcggag gaggagcagc gcgggagcca    240
   ggcgctgccc caagaccctg cctgcgtccg agcgagcgga acctcgcgct tcgcccgggg    300
   acaatccgaa gtccgcgcta tggaagagga gaaatatttg cctgagctga tggcagagaa    360
   agatagcctg gatccatctt ttgtgcatgc gtcgcgcctt ttggcagaag gtaggacttg    420
   cc                                                                   422
<210> 16
   <211> 339
   <212> DNA
   <213> Homo sapiens
<220>
   <223> ASCL2_MA_8
<400> 16
   gctaacaaag ctgggttcct gctgggcccc gccctgctcc tcgcccccgc gactgggctg     60
   ggcgcgctgt cccctagcgc agctatgtcc cgagcgcgcc cccacctgtg cgttaatcta    120
   ctgggaatgg gggtggactg cgccttacct ggggcggggt ggggcttaag gagtggtcga    180
   gactgaggcg gggtgggagg ttcaggttcc cggggcgcct tccccaaccc gccccgcttt    240
   ccccgtccct ccacgcgcac cctgcctgtg gtttccgtgc gcccccggcc tgagggctct    300
   gggcggcacc ttaacccgga gggcctggag gtctgcacc                           339
<210> 17
   <211> 38
   <212> DNA
   <213> Artificial
<220>
   <223> Artificially synthesized primer sequence (SLC13A5_MA_10 forward p rimer for using MassARRAY assay)
<400> 17
   aggaagagag gaaggatttg aatttggaga tatagttt 38
<210> 18
   <211> 56
   <212> DNA
   <213> Artificial
<220>
   <223> Artificially synthesized primer sequence (SLC13A5_MA_10 reverse p rimer for using MassARRAY assay)
<400> 18
   cagtaatacg actcactata gggagaaggc taaaaaaccc aaaaacctac aaaaaa 56
<210> 19
   <211> 32
   <212> DNA
   <213> Artificial
<220>
   <223> Artificially synthesized primer sequence (SLC13A5_MA_13 forward p rimer for using MassARRAY assay)
<400> 19
   aggaagagag tttttttggg ttttgaaggg tt 32
<210> 20
   <211> 57
   <212> DNA
   <213> Artificial
<220>
   <223> Artificially synthesized primer sequence (SLC13A5_MA_13 reverse p rimer for using MassARRAY assay)
<400> 20
   cagtaatacg actcactata gggagaaggc tttatatccc ttcctctcta aaactcc 57
<210> 21
   <211> 32
   <212> DNA
   <213> Artificial
<220>
   <223> Artificially synthesized primer sequence (SLC13A5_MA_15 forward p rimer for using MassARRAY assay)
<400> 21
   aggaagagag ttttttttgt tttaggggtt gt 32
<210> 22
   <211> 55
   <212> DNA
   <213> Artificial
<220>
   <223> Artificially synthesized primer sequence (SLC13A5_MA_15 reverse p rimer for using MassARRAY assay)
<400> 22
   cagtaatacg actcactata gggagaaggc tccaccaaca taaataaaac tcccc 55
<210> 23
   <211> 34
   <212> DNA
   <213> Artificial
<220>
   <223> Artificially synthesized primer sequence (FAM150A_MA_14 forward p rimer for using MassARRAY assay)
<400> 23
   aggaagagag gggaggattt agtagggtaa ttgt 34
<210> 24
   <211> 56
   <212> DNA
   <213> Artificial
<220>
   <223> Artificially synthesized primer sequence (FAM150A_MA_14 reverse p rimer for using MassARRAY assay)
<400> 24
   cagtaatacg actcactata gggagaaggc ttttcaccta aaaaaacact aaaacc 56
<210> 25
   <211> 36
   <212> DNA
   <213> Artificial
<220>
   <223> Artificially synthesized primer sequence (GRM6_MA_8 forward prime r for using MassARRAY assay)
<400> 25
   aggaagagag ggtttaggat aagtttgtga tagatg 36
<210> 26
   <211> 56
   <212> DNA
   <213> Artificial
<220>
   <223> Artificially synthesized primer sequence (GRM6_MA_8 reverse prime r for using MassARRAY assay)
<400> 26
   cagtaatacg actcactata gggagaaggc taaaacaaaa aaacaaaccc aaaaat 56
<210> 27
   <211> 33
   <212> DNA
   <213> Artificial
<220>
   <223> Artificially synthesized primer sequence (ZFP42_MA_2 forward prim er for using MassARRAY assay)
<400> 27
   aggaagagag gagttgatgg gtggttgtag ttt 33
<210> 28
   <211> 60
   <212> DNA
   <213> Artificial
<220>
   <223> Artificially synthesized primer sequence (ZFP42_MA_2 reverse pri mer for using MassARRAY assay)
<400> 28
   cagtaatacg actcactata gggagaaggc tcccatttaa aaaaaattcc ataaaacaaa 60
<210> 29
   <211> 40
   <212> DNA
   <213> Artificial
<220>
   <223> Artificially synthesized primer sequence (ZNF154_MA_5 forward pri mer for using MassARRAY assay)
<400> 29
   aggaagagag ggtgaatata ttttagagaa gttaaaatgg 40
<210> 30
   <211> 56
   <212> DNA
   <213> Artificial
<220>
   <223> Artificially synthesized primer sequence (ZNF154_MA_5 reverse pri mer for using MassARRAY assay)
<400> 30
   cagtaatacg actcactata gggagaaggc ttccctccac taccctaaaa cttaaa 56
<210> 31
   <211> 35
   <212> DNA
   <213> Artificial
<220>
   <223> Artificially synthesized primer sequence (RIMS4_MA_9 forward prim er for using MassARRAY assay)
<400> 31
   aggaagagag ggagttttag tttatgaggg aagga 35
<210> 32
   <211> 54
   <212> DNA
   <213> Artificial
<220>
   <223> Artificially synthesized primer sequence (RIMS4_MA_9 reverse prim er for using MassARRAY assay)
<400> 32
   cagtaatacg actcactata gggagaaggc taaaccccaa aatctccaaa atac 54
<210> 33
   <211> 37
   <212> DNA
   <213> Artificial
<220>
   <223> Artificially synthesized primer sequence (TRH_MA_8 forward primer for using MassARRAY assay)
<400> 33
   aggaagagag aatagatttt tagaggtggt gtagaaa 37
<210> 34
   <211> 55
   <212> DNA
   <213> Artificial
<220>
   <223> Artificially synthesized primer sequence (TRH_MA_8 reverse primer for using MassARRAY assay)
<400> 34
   cagtaatacg actcactata gggagaaggc taaaaaactc cctttccaat actcc 55
<210> 35
   <211> 37
   <212> DNA
   <213> Artificial
<220>
   <223> Artificially synthesized primer sequence (ZNF540_MA_17 forward pr imer for using MassARRAY assay)
<400> 35
   aggaagagag gggtagggta gaattaggtt aaagaaa 37
<210> 36
   <211> 56
   <212> DNA
   <213> Artificial
<220>
   <223> Artificially synthesized primer sequence (ZNF540_MA_17 reverse pr imer for using MassARRAY assay)
<400> 36
   cagtaatacg actcactata gggagaaggc tactaaaatc aataaccccc aaaaaa 56
<210> 37
   <211> 35
   <212> DNA
   <213> Artificial
<220>
   <223> Artificially synthesized primer sequence (PCDHAC1_MA_5 forward pr imer for using MassARRAY assay)
<400> 37
   aggaagagag tggtagtttt tgggatataa gaggg 35
<210> 38
   <211> 56
   <212> DNA
   <213> Artificial
<220>
   <223> Artificially synthesized primer sequence (PCDHAC1_MA_5 reverse pr imer for using MassARRAY assay)
<400> 38
   cagtaatacg actcactata gggagaaggc taaactaccc aaatcttaac ctccac 56
<210> 39
   <211> 37
   <212> DNA
   <213> Artificial
<220>
   <223> Artificially synthesized primer sequence (PRAC_MA_2 forward prime r for using MassARRAY assay)
<400> 39
   aggaagagag ggtgaaagtt tgttgtttat ttttttt 37
<210> 40
   <211> 56
   <212> DNA
   <213> Artificial
<220>
   <223> Artificially synthesized primer sequence (PRAC_MA_2 reverse prime r for using MassARRAY assay)
<400> 40
   cagtaatacg actcactata gggagaaggc tcaaactaaa ttctaatccc cacctt 56
<210> 41
   <211> 35
   <212> DNA
   <213> Artificial
<220>
   <223> Artificially synthesized primer sequence (ZNF671_MA_8 forward pri mer for using MassARRAY assay)
<400> 41
   aggaagagag tgggatatag gggttgtagg tattt 35
<210> 42
   <211> 56
   <212> DNA
   <213> Artificial
<220>
   <223> Artificially synthesized primer sequence (ZNF671_MA_8 reverse pri mer for using MassARRAY assay)
<400> 42
   cagtaatacg actcactata gggagaaggc tataaaaacc acactctacc cacaaa 56
<210> 43
   <211> 35
   <212> DNA
   <213> Artificial
<220>
   <223> Artificially synthesized primer sequence (WNT3A_MA_9 forward prim er for using MassARRAY assay)
<400> 43
   aggaagagag gtttatttgg taatgagggg ttgtt 35
<210> 44
   <211> 56
   <212> DNA
   <213> Artificial
<220>
   <223> Artificially synthesized primer sequence (WNT3A_MA_9 reverse prim er for using MassARRAY assay)
<400> 44
   cagtaatacg actcactata gggagaaggc tttcctcaat cttaaacatc tcaaaa 56
<210> 45
   <211> 38
   <212> DNA
   <213> Artificial
<220>
   <223> Artificially synthesized primer sequence (KHDRBS2_MA_19(rev) forw ard primer for using MassARRAY assay)
<400> 45
   aggaagagag tttggtatta ttattaatga gtggttgg 38
<210> 46
   <211> 57
   <212> DNA
   <213> Artificial
<220>
   <223> Artificially synthesized primer sequence (KHDRBS2_MA_19(rev) reve rse primer for using MassARRAY assay)
<400> 46
   cagtaatacg actcactata gggagaaggc taacaaatcc taccttctac caaaaaa 57
<210> 47
   <211> 35
   <212> DNA
   <213> Artificial
<220>
   <223> Artificially synthesized primer sequence (ASCL2_MA_8 forward prim er for using MassARRAY assay)
<400> 47
   aggaagagag gttaataaag ttgggttttt gttgg 35
<210> 48
   <211> 53
   <212> DNA
   <213> Artificial
<220>
   <223> Artificially synthesized primer sequence (ASCL2_MA_8 reverse prim er for using MassARRAY assay)
<400> 48
   cagtaatacg actcactata gggagaaggc taatacaaac ctccaaaccc tcc 53
<210> 49
   <211> 384
   <212> DNA
   <213> Homo sapiens
<220>
   <223> FAM150A_MA_14
<400> 49
   gggaggaccc agtagggtaa ctgctgtgtt gccctggtgg ttctccctgg gctctgtctc      60
   ctgctgcctc caccccctga gccttggggt ctgtcatggc ttcccctggc tggtggggtc     120
   agtagaacct gtggtgccta ggtctggatg gaaaaaagca gggctggggt gtggcctgga     180
   tgagtggaga tctctgtgcc ttgggctcaa aggtgtgggg tgtgctctgc tgctgagccc     240
   ctgcttgctc aggaacactg gccatgctgt catgccagct gcccctgccc caggtctgga     300
   ggcctgacct gctctcctag gtgcagcact gtgttctctt ctgtgtgggg gagtggtggg     360
   tggaagaggt ctggggctgg gcac                                             384
<210> 50
   <211> 384
   <212> DNA
   <213> Homo sapiens
<220>
   <223> FAM150A_MA_14
<400> 50
   gggaggaccc agtagggtaa ctgccgcgtc gccccggcgg ttctccctgg gctctgtctc      60
   ccgccgcctc caccccccga gcctcggggt ccgtcacggc ttcccctggc tggcggggtc     120
   agtagaaccc gcggcgccta ggtccggacg gaaaaaagca gggccggggt gcggcctgga     180
   tgagcggaga tctccgcgcc ttgggctcaa aggtgcgggg tgcgctctgc tgccgagccc     240
   ctgctcgctc aggaacactg gccacgccgt cacgccagcc gcccctgccc caggtctgga     300
   ggcccgacct gctctcctag gcgcagcacc gcgttctctt ccgcgtgggg gagcggcggg     360
   cggaagaggt ctggggctgg gcac                                             384
<210> 51
   <211> 24
   <212> DNA
   <213> Oligonucleotide primer
<400> 51
   gggaggattt agtagggtaa ttgt 24
<210> 52
   <211> 22
   <212> DNA
   <213> Oligonucleotide primer
<400> 52
   atacccaacc ccaaacctct tc 22